# EUROPEAN PATENT SPECIFICATION

(11) **EP 4 132 367 B1**
(45) Date of publication and mention of the grant of the patent: **14.10.2026**
(21) Application number: 20719144.6
(22) Date of filing: 08.04.2020
(51) Int. Cl.: A61B 10/04, A61B 10/02

(54) **KIT OF PARTS**
TEILESATZ
KIT DE PIÈCES

(43) Date of publication of application: 15.02.2023
(73) Proprietor: BibbInstruments AB, 223 63 Lund (SE)
(72) Inventor: WALTHER, Charles, 223 58 Lund (SE); DYMLING, Stephan, 216 22 Limhamn (SE); WALTHER, Bruno, 241 62 Löberöd (SE)
(74) Representative: AWA Sweden AB
(86) International application number: PCT/EP2020/060041
(87) International publication number: WO 2021/204376

(56) References cited:
- WO-A1-2013/155557
- WO-A1-2016/054063
- WO-A1-2016/154170
- WO-A1-2019/155472
- WO-A2-93/07819
- US-A1- 2009 118 641

## Description

### Field of invention

The invention relates to a kit of parts comprising a biopsy instrument and a manoeuvring unit.

### Technical Background

A biopsy is a medical test commonly performed by a physician involving sampling of cells or tissues for examination. The biopsy is often acquired using a biopsy instrument inserted into a patient's body via an endoscope. A large variety of endoscopic biopsy instruments are commercially available today, the majority of which are biopsy forceps that pinch off the tissue sample, or fine needles that aspirate cells by applying under-pressure.

For some diagnostic purposes, millimetre-sized samples retrievable using said biopsy forceps are sufficient, but for some types of lesions and tumours, such as comparably deep lesions or deep growing tumours, such small and superficial millimetre-sized samples are inadequate for making a diagnosis. The fine needles are often capable of reaching deeper tumours but are only capable of retrieving small amounts of dispersed cells, thereby limiting the diagnostic ability.

When taking a tissue sample with an endoscopic biopsy instrument, the instrument is inserted in a working channel of an endoscope, and advanced to the biopsy site. After the tissue sample has been obtained, the endoscopic biopsy instrument is retracted from the endoscope such that the tissue sample can be placed in a storage unit for evaluation by a pathologist.

Biopsy is today the primary diagnostic tool for determining malignancy of neoplastic growths. As the methods of cancer treatment have been improved and refined, the number of biopsies required for diagnostics have increased. Before the optimal method of treatment can be determined, the spread and density of the malignant cells need to be assessed, which in for example the diagnostics of laryngeal or esophageal cancer may require 20 - 30 biopsies, a process which is time consuming and incommodious for both the patient and the physician. Apart from that, the biopsy forceps separate the tissue sample from the body of the patient by tearing, which risks damaging the tissue sample and makes it more difficult to evaluate the tissue sample. The fine needles supply small amounts of cells that cannot be prepared by routine histological methods and typically also require more advanced endoscopic equipment with ultrasound.

In this context one may mention WO201166470 which discloses an endoscopic biopsy instrument having a kind of forceps with a storage lumen for multiple biopsies. The biopsies are transported upwardly into the storage lumen by the use of a suction which is applied when a sample is retrieved.

Another technology sometimes used is the provision of a needle having a closed distal end and instead having an opening in the circumferential surface close to the distal end. In such needles there is made use of a suction which sucks a portion of the tissue into the opening in the circumferential surface. Inside the needle there is provided a reciprocating cutting tool which passes back and forth past the opening and cuts the portion of the tissue being inside the circumferential surface. Examples of this technology are e.g. shown in US20100152756 and US20060074343.

In WO200197702 there is disclosed a biopsy instrument in which an outer needle or cannula is inserted into a tissue and brought into contact with a lesion whereby a continuous suction applied at the proximal end of the cannula is used to fixate the lesion to the distal end of the cannula. While maintaining a suction force keeping the lesion in place, a second medical device, such as a biopsy needle or a cryoprobe, is inserted through an airtight seal at the proximal end of the instrument and through the cannula to the lesion. In US 2013/0223702 A1 there is also disclosed various kinds of biopsy instruments using forceps, an auger or vacuum to draw a tissue sample into the instrument.

In WO2019/155472 is considered the closest prior art, and there is disclosed and claimed a soft tissue biopsy device comprising; an elongated handle comprising a gripping member; an elongated flexible shaft mechanically comprising a hollow distal sampling portion with an internal lumen and a distal opening facing a soft tissue; a driving unit configured to rotate said sampling portion with a speed in a range of 100 rounds-per-minute (RPM) to 12,000 RPM while said sampling portion axially advances into said soft tissue.

In WO2016/054063 A1 there is disclosed an endoscope for removing tissue at a surgical site, which endoscope includes an elongated tubular body insertable within a mammalian cavity of a patient, wherein an instrument channel extends between a first opening at a distal end and a second opening at a proximal end of the tubular body and is sized an configured to receive a surgical cutting assembly that includes an aspiration channel configured to remove material entering the endoscope via distal end of the surgical cutting assembly.

US2009/0118641 A1 discloses biopsy devices each comprising of tissue collection element having a distal end a proximal end connected to a drive mechanism. The tissue collection element is translationally and rotationally moveable within a target location in response to actuation by the drive mechanism, thereby collecting tissue.

WO93/07819 discloses a device to excise tissue samples from a living body including a means to prevent lateral movement of the cutting edge as it engages the tissue and a means to control the depth of excision. The device also features an external sheath which is driven into the tissue with the coring shaft and which provides the operator with continued access to the sampling site after removal of the tissue core.

A problem with the above disclosed technologies is also that they rely on the application of a suction, which renders the instrument complicated.

It would therefore be advantageous to have a biopsy instrument which allows for a straight-forward and robust design and which is capable of retrieving tissue samples in an amount being sufficient for diagnostics in a short time. In addition, it would be advantageous if the biopsy instrument provided tissue samples which are coherent.

### Summary of invention

It is an object of the invention to provide a biopsy instrument which allows for a straight-forward and robust design and which is capable of retrieving tissue samples in an amount being sufficient for diagnostics in a short time.

This object has been achieved by a kit of parts comprising
a biopsy instrument, and
a manoeuvring unit comprising a motor,
   wherein the biopsy instrument comprises
      an outer elongated hollow tubular member which extends from a proximal end to a distal end along a central geometrical axis, and
      a base member which extends from a proximal end to a distal end along the central geometrical axis, wherein at least a distal end portion of the base member is shaped as an elongated hollow tube, the elongated hollow tube at the distal end of the base member being intended to be at least partly inserted into a tissue from which a biopsy is to be obtained,
      wherein the base member is arranged inside the outer elongated hollow tubular member and is independently rotationally and translationally movable relative to the outer elongated hollow tubular member,
      wherein the base member is capable of transferring a force along the central geometrical axis such that a movement of the proximal end of the base member along the central geometrical axis is transferred to a movement of the distal end of the base member along the central geometrical axis, and of transferring a torque about the central geometrical axis such that a rotation and a torque applied by a motor at the proximal end of the base member about the central geometrical axis is transferred from the proximal end of the base member to the distal end of the base member thereby rotating the distal end of the base member about the central geometrical axis,
      wherein the elongated hollow tube is capable of being advanced out of a distal end of the outer elongated hollow tubular member and to be retracted back into the outer elongated hollow tubular member by a movement of the proximal end of the base member along the central geometrical axis, while being rotated inside and relative to the outer elongated hollow tubular member about the central geometrical axis by the motor applying a rotation and a torque at the proximal end of the base member,
      wherein the elongated hollow tube is provided with a distally facing circular cutting edge defining a mouth of the distal end of the elongated hollow tube, and
      wherein the elongated hollow tube has, at a distal portion of the elongated hollow tube, a hollow elongated tubular sample acquiring portion having a smooth interior surface,
   wherein the proximal end of the base member is configured to be connected to the motor such that rotation and torque may be applied by the motor to the proximal end of the base member and be transferred by the base member to the elongated hollow tube at the distal end of the base member, and
   wherein the motor is configured to provide a rotation of the elongated hollow tube, at a rotational speed of at least 13 000 rpm, while the elongated hollow tube is advanced out of the distal end of the outer elongated hollow tubular member and is retracted back into the outer elongated hollow tubular member, inside and relative to the outer elongated hollow tubular member about the central geometrical axis by applying rotation and torque to the proximal end of the base member, the base member being flexible and the outer elongated hollow tubular member being flexible.

The kit of parts is advantageous compared to prior art biopsy instruments in that it makes it possible to retrieve tissue samples in an amount being sufficient for diagnostics in a comparably short time. The kit of parts may also be referred to as a biopsy instrument or instrument. In such an instant the part mentioned as biopsy instrument in the above may e.g. be referred to as a disposable part of the biopsy instrument. The kit of parts may also be referred to as a biopsy system. The instrument is capable of retrieving a plurality of tissue samples directly one after the other without a previous sample needs to be harvested.

When a biopsy is to be acquired, the cutting edge and the distal end of the elongated hollow tube is configured to be advanced along the central geometrical axis into a tissue while being rotated by being motor driven at its proximal end at a rotational speed of at least 13 000 rpm and thereby cut a core of the tissue which, due to the advancement of the elongated hollow tube, enters relative to the elongated hollow tube through the mouth into the sample acquiring portion of the elongated hollow tube with a circumferential outer surface of the core at least partly abutting the smooth interior surface of the sample acquiring portion, where-after the elongated hollow tube is retracted from the tissue while being rotated at a rotational speed of at least 13 000 rpm by being motor driven at its proximal end whereby the core of the tissue is detached from the tissue by a pulling force due to the retraction of the elongated hollow tube and due to an adhesive force formed at an interface between the smooth interior surface and the circumferential outer surface of the core which force keeps the core inside the sample acquiring portion having the smooth interior surface.

A first sample is in a controlled manner pushed further into the hollow tube towards the proximal end by the core of the second sample when the distal end is advanced into the tissue for a second time. The fact that the hollow tube is provided with a smooth interior surface makes the core, due to the smooth surface and the presence of liquid in the tissue, to become adhered to the inside of the hollow tube, which makes it possible to retrieve samples with a minimum of damage to the sample and still allow for the cutting edge and distal end to be drilled into and out of the tissue thereby reducing discomfort for the patient. As the core becomes adhered to the inside of the elongated tubular member, the core will at the mouth of the elongated tubular member be twisted and be released from the sample site by shearing and/or tensile forces. Compared to prior art biopsy instruments there is with the inventive biopsy instrument no need for any hooks or the like on the inside of the instrument, which hooks has the drawback that they are difficult to combine with drilling in and out of the tissue and still avoiding damaging the samples. The fact that the inventive biopsy instrument is so gentle to the samples also allows for the samples to be harvested in a controlled manner such that each sample is still uniquely identifiable and still undamaged or coherent. This allows for the physician to keep any information provided by the stratigraphy and/or position of respective sample, which in turn may be used to increase the amount of data provided by the biopsy, which in turn may increase the accuracy of the diagnosis ultimately provided.

It may be noted that the wording that the base member is independently rotationally and translationally movable relative to the outer elongated hollow tubular member is intended to refer to the fact that the rotational movement is independent from the translational movement and vice versa.

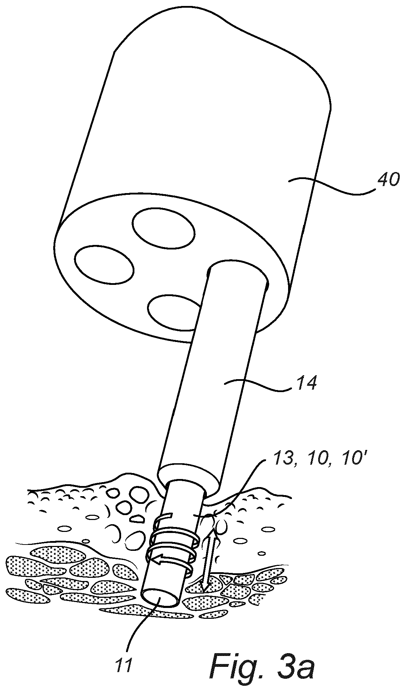

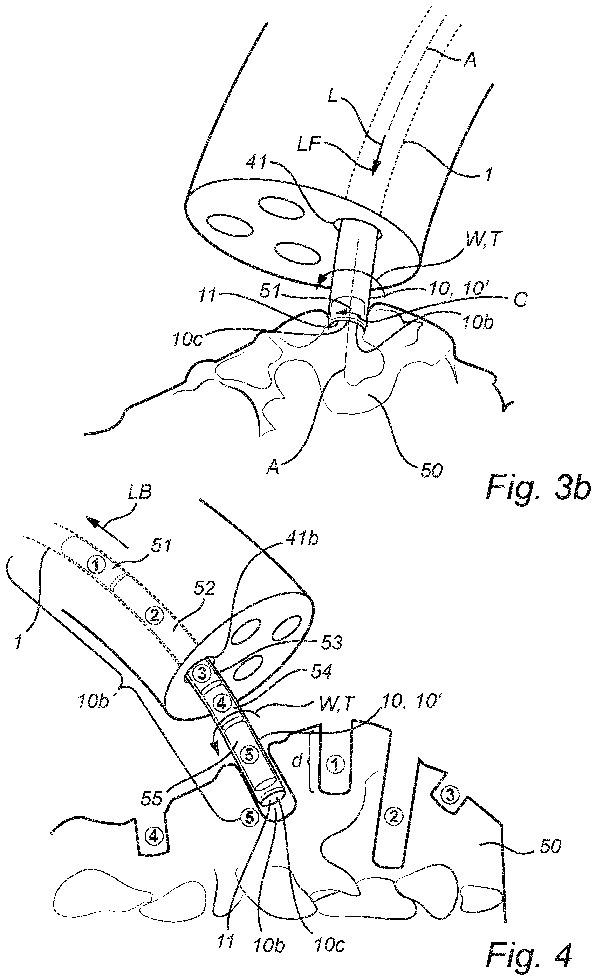

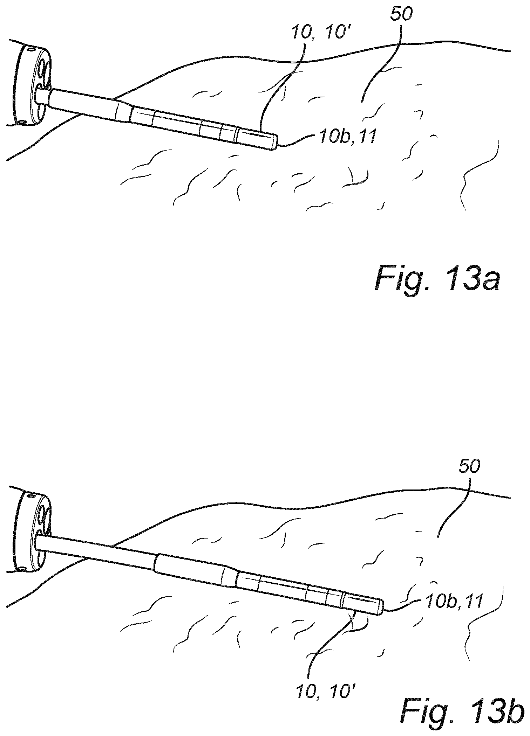

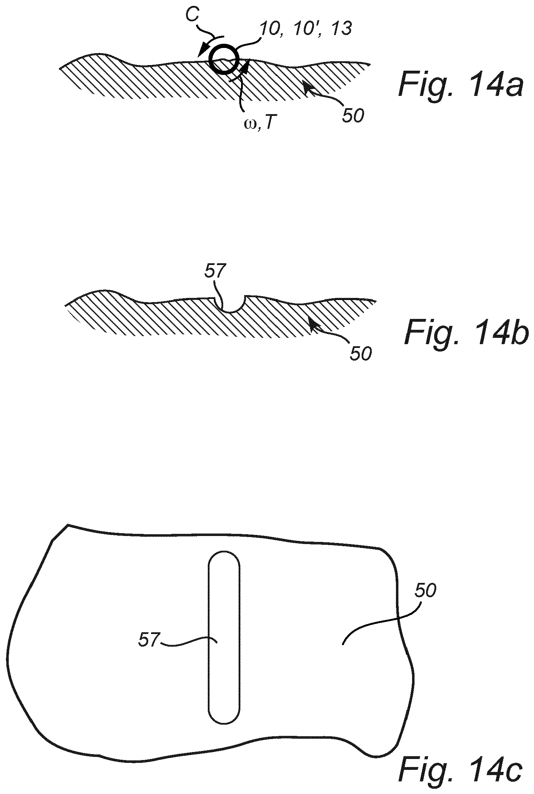

It may be noted that in the above it is referred to a reference sample. This notion of referring to a reference sample when defining a reference for the smoothness is used since the biopsy instrument may in actual biopsy sampling be used in accordance with a number of different methods. It may e.g. be used in accordance with one method where the biopsy instrument is actually used as referred to in the above and as e.g. shown in figures 3-5, i.e. where the distal end is advanced a distance into the tissue and thereafter is retraced. However, the biopsy instrument may in accordance with another method be used to move along the surface of the tissue from which the biopsy is to be obtained as e.g. shown in figures 13a-c and 14a-c. In the user method shown in figures 3 and 4, the distal end is fully inserted into the tissue in the sense that the distal end is inserted with the complete circumference inserted into the tissue whereby an adhesive force larger than breaking force needed to detach the core from the tissue is formed. In the method shown in figures 13a-c and 14a-c, the distal end is only partly inserted into the tissue in the sense that the distal end is inserted with only a portion of the complete circumference being inserted into the tissue. The smoothness of the surface has advantages in both methods, but the adhesive force provided by the smoothness is clearly pronounced and observable by the detachment of the core from the remainder of the tissue when performing the reference sample as referred to above. It may be noted that the reference sample refers to a sample performed in healthy tissue.

The hollow tube has preferably an extension, i.e. a length along the central geometrical axis, and is provided with said smooth surfaces along a length from the distal end towards the proximal end, the extension having at least a length allowing for at least two, preferably at least three, reference samples of the above disclosed kind to be acquired one after the other.

The base member and outer elongated hollow tubular member are from a bending perspective flexible whereby the biopsy instrument is capable of extending along a central geometrical axis having various and over time varying shapes, which is typically required for a biopsy instrument for use in an endoscope. Such a flexible biopsy instrument for use in an endoscope is sometimes referred to as an endoscopic biopsy instrument. Providing rotational speed of at least 13 000 rpm is especially useful for a design where the base member and outer elongated hollow tubular member are from a bending perspective flexible, since the comparably high rotational speed will allow a comparably blunt cutting edge to effectively penetrate the tissue and since the rotation of the base member will stabilise the part of the distal end of the base member extending out of the outer elongated hollow tubular member. The fact that it is possible to have a comparably blunt cutting edge is advantageous since it reduces the risk that the cutting edge gets stuck inside the outer elongated hollow tubular member, especially when the outer elongated hollow tubular member is flexible and is comparably sharply bent. Preferably, the cutting edge is a blunt cutting edge. The fact that it is possible to have a comparably blunt cutting edge is advantageous since it reduces the risk that the cutting edge accidentally cuts the tissue before the rotation is activated. The rotational speed may according to a preferred embodiment be between 13 000 rpm and 25 000 rpm. The upper limit is amongst others dependent upon mechanical constraints. A higher upper limit, such as 30 000 rpm, may be possible in some embodiments. Moreover, an improved effect is presently not obtained with higher rotational speeds in the embodiments described herein but may be achieved in other designs or embodiments of the invention. In accordance with a more preferred embodiment, the rotational speed is between 13 000 rpm and 20 000 rpm. It may be noted that in order to achieve the desired cutting effect, the rotational speed of at least 13 000 rpm is relative to the tissue. However, from a practical standpoint, since the outer elongated hollow tubular member is connected to a housing of the manoeuvring unit and the base member is connected to the motor, the rotational speed of the base member is also at least 13 000 rpm relative to the outer elongated hollow tubular member.

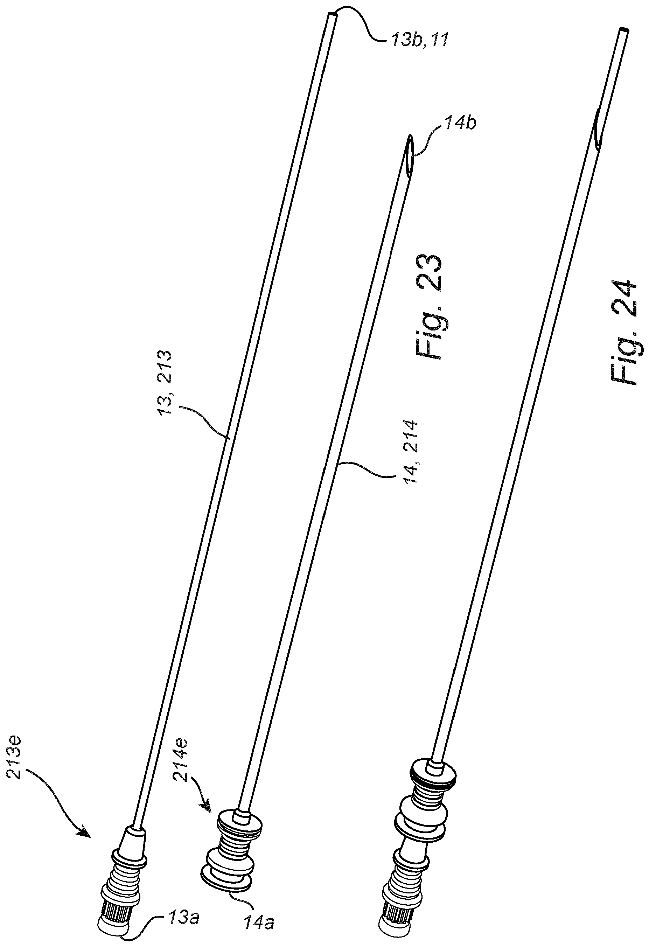

It may be noted that, in accordance with an alternative, the base member and the outer elongated hollow tubular member may from a bending perspective be rigid and extend with the central geometrical axis extending along a straight line. Such a rigid instrument is typically used as a separate biopsy instrument. Thus, it is typically not used in combination with an endoscope. An example of such an instrument is shown in figures 23-25 and 26a-b. It may in this context be noted that for such an instrument, it is conceivable to use a lower rotational speed compared to the one used for the flexible instrument. Thus, the base member and the outer elongated hollow tubular member may be rigid, and the motor is designed to provide a rotational speed of at least 3 000 rpm.

It may be noted that the outer elongated hollow tubular member is connected to a part of the manoeuvring unit other than to the motor, such that the base member may be rotated by the motor relative to the outer elongated hollow tubular member. The outer elongated hollow tubular member may e.g. be connected to the housing or be connected to the housing via a telescopic mechanism. The telescopic mechanism allows the outer elongated hollow tubular member to be translationally moved relative to the housing within boundaries defined by the telescopic mechanism. It may be noted that the telescopic mechanism may allow the outer elongated hollow tubular member to be rotated relative to the housing, such as being rotated by hand allowing the angular orientation of the outer elongated hollow tubular member to be adjusted. Such adjustment could e.g. be of interest if the distal end of the outer elongated hollow tubular member is provided with a stopper having a varying shape as seen along the circumference of the outer elongated hollow tubular member. However, the proximal end of the outer elongated hollow tubular member is connected to the manoeuvring unit such that it is at least semi-stationary relative to the housing, i.e. such that it is not rotated by any motor relative to the housing. It is advantageous that the outer elongated hollow tubular member is stationary relative to the tissue such that it does not accidentally damage the tissue, which could otherwise easily happen if the outer elongated hollow tubular member would be rotating at high speed relative to the tissue. It may in this context be noted that it is advantageous that the instrument allows the elongated hollow tube of the base member to be rotated with the high rotational speed before it is advanced out of the outer elongated hollow tubular member, while it is advanced into the tissue and also while it is retracted back into the outer elongated hollow tubular member. It may be noted that it is conceivable to also provide a removable inner member being configured to be positioned inside the base member and being configured to, during insertion of the biopsy instrument, close the opening of the hollow tube at the distal end of the base member, thereby preventing unwanted filling of tissue into the hollow tube. The removable inner member may also be configured to close of the distal opening of the outer elongated hollow member during insertion of the biopsy instrument, thereby preventing unwanted filling of tissue into the outer elongated hollow member.

It may be noted that the rotational direction during advancement and retraction may, but need not, be the same. It is e.g. advantageous to have the same rotational direction e.g. in case the base member is stronger in transferring a torque in one rotational direction compared to its capability of transferring a torque in the opposite rotational direction. Such difference in torque transferring capability may e.g. occur in case the base member is designed as a wire, such as a wire rope or a hollow wire rope. In one rotational direction, the windings in the wire has a tendency to tighten and the wire is typically comparably strong when transferring a torque having a tendency to tighten the windings. It is from a user perspective advantageous if the rotation is maintained in the same rotational direction, and preferably also at the same or at least similar rotational speed, during the advancement of the elongated hollow tube of the base member into the tissue and during the retraction of the elongated hollow tube of the base member out of the tissue, since any tactile feedback from the tissue, via the instrument, to the user will typically originate from the tissue and will not be influenced by a difference in the specific interaction between the tissue and the elongated hollow tube for different rotational directions and/or rotational speeds.

The interior or outer surface of the sample acquiring portion is preferably liquid tight. This is beneficial when it comes to the forming of an adhesion force between the outer envelope surface of the tissue core during the retraction of the elongated hollow tube out of the tissue. The change in geometry of the tissue when subjected to the pulling force will in a sense locally result in a local under-pressure reinforcing the adhesion. This occurrence of a local under-pressure is especially pronounced if the elongated hollow tube is closed at the proximal end or subjected to an under-pressure at the proximal end. Preferably, the interior or outer surface of the sample acquiring portion is also gas tight. It may be noted that the preferred property of the interior or outer surface being liquid tight and more preferred also gas tight does not necessarily mean that the interior or outer surface needs to be liquid tight and gas tight when it comes to long term performance. The preferred property of the interior or outer surface being liquid tight refers to the fact that the surface is preferably liquid tight at least for the time period sufficient to retrieve the biopsy samples and preferably also sufficient to allow the samples to be harvested. That is, the interior or outer surface should preferably be liquid tight for at least several seconds. Similarly, it is preferred that the interior or outer surface is also gas tight at least for the time period sufficient to retrieve the biopsy samples and preferably also sufficient to allow the samples to be harvested.

In the preferred embodiments, the inner surface is liquid tight and more preferred also gas tight. It may be noted that preferably, the smooth inner surface is liquid tight and more preferred also gas tight.

According to a preferred embodiment, the distally facing circular cutting edge is shaped and the smooth interior surface, preferably being a liquid tight smooth interior surface, connects to the cutting edge such that the smooth interior surface, preferably being a liquid tight smooth interior surface, extends, as seen along the central geometrical axis, to a most distal part of the cutting edge. The fact that the smooth interior surface connects to the cutting edge such that the smooth interior surface extends, as seen along the central geometrical axis, to a most distal part of the cutting edge provides an adhesion between the smooth interior surface and the circumferential outer surface of the core all the way to the cut in the tissue as seen along the central geometrical axis. This is beneficial when it comes to retrieving a distinct and efficient separation between the core of tissue caught inside the elongated hollow tube and the remaining part of the tissue just outside the mouth of the sample acquiring portion of the elongated hollow tube. This is further enhanced if the smooth interior surface is a liquid tight smooth interior surface connecting to the cutting edge such that the liquid tight smooth interior surface, extends, as seen along the central geometrical axis, to a most distal part of the cutting edge.

The smooth interior surface is preferably smooth to such an extent that when a reference biopsy is to be acquired, the cutting edge and the distal end of the elongated hollow tube is configured to be advanced along the central geometrical axis into a tissue while being rotated by being motor driven at its proximal end at a rotational speed of at least 13 000 rpm and thereby cut a core of the tissue which, due to the advancement of the elongated hollow tube, enters relative to the elongated hollow tube through the mouth into the sample acquiring portion of the elongated hollow tube with a circumferential outer surface of the core at least partly abutting the smooth interior surface of the sample acquiring portion, where-after the elongated hollow tube is retracted from the tissue while being rotated at a rotational speed of at least 13 000 rpm by being motor driven at its proximal end whereby the core of the tissue is detached from the tissue by a pulling force due to the retraction of the elongated hollow tube and due to an adhesive force formed at an interface between the smooth interior surface and the circumferential outer surface of the core which force keeps the core inside the sample acquiring portion having the smooth interior surface.

The surface is preferably smooth to such an extent that when performing a reference sample with a biopsy instrument of the above kind, a core is, during retraction of the elongated hollow tube, detached from the tissue in case the distal end has been inserted into the tissue a distance being the same or greater than an inner diameter of the mouth. However, it is in many cases preferred that the surface is smooth to such an extent that when performing a reference sample with a biopsy instrument of the above kind, a core is, during retraction of the hollow tube, detached from the tissue in case the distal end has been inserted into the tissue a distance being 1,3 times or greater than an inner diameter of the mouth. However, it is in many more preferred that the surface is smooth to such an extent that when performing a reference sample with a biopsy instrument of the above kind, a core is, during retraction of the hollow tube, detached from the tissue in case the distal end has been inserted into the tissue a distance being at least 1,7 times or greater than an inner diameter of the mouth. The above applies at least for inner diameters being between 1-5mm.

The smooth inner surface has preferably a surface roughness with an Ra value of less than 1,5µm, preferably less than 1 µm, when formed of steel, such as a medical grade stainless steel, and less than 6 µm, such as between 1 µm and 6 µm, when formed of a polymer-based material. The smooth inner surface has preferably a surface roughness with an Ra value between 0,05 and 1,5µm, preferably between 0,05 and 1µm, when formed of steel, such as a medical grade stainless steel. The surface roughness value Ra is preferably determined according to standard ISO 4287:1997. Preferably, the surface has a low friction. This is presently considered to be one reason behind that it is possible to have a higher Ra-value when the smooth inner surface is formed of a polymer-based material compared to when the smooth inner surface is formed of steel. Thus, it is considered that suitable Ra-values increases with decreasing friction coefficient. Thus, it is found that one suitable combination is a material having a friction coefficient of about 0,6-1,0, such as for steel against steel, is combined with a Ra value between 0,05 and 1,5µm, preferably between 0,05 and 1µm, and that another suitable combination is a material having a friction coefficient of about 0,02-0,3, such as for polymer-based materials mentioned below, is combined with a Ra value between 1 µm and 6 µm.

The smooth surface is arranged inside a tubular member, such as the elongated hollow tube, having parallel and straight generatrixes and being circular in cross-section. The smooth surface is arranged without protrusions. The smooth surface is arranged to cover the entire inner periphery of the tubular member. The smooth surface is arranged at least at a distal end of the tubular member.

The tubular member may also have an outer surface having a predetermined smoothness, at least on a distal portion of the tubular member.

The smooth surface may be the inner surface of the tubular member. The inner surface of the tubular member may be machined to a predetermined smoothness. The inner surface may be medical grade stainless steel.

The smooth surface may be a layer or film arranged on the inner surface of the tubular member.

The smooth surface is arranged along said tubular member at least over a length corresponding to a sample to be obtained. A length of the sample may be a few millimetres up to about 50 mm. The smooth surface may be arranged along said tubular member over a length corresponding to several consecutive samples to be obtained in sequence. The smooth surface may extend up to the cutting edge or end a short distance from the cutting edge, such as 0.5 mm from the cutting edge.

It is e.g. conceivable to form the base member such that it at its distal end comprises an endtube, such as a rigid endtube, formed of steel being machined such that the inside of the endtube is formed of steel and is smooth to the extent discussed above in order to be able to acquire a sample, wherein the endtube is relatively short to allow the instrument to follow bends of the endoscope. Proximal of this endtube, the base member is formed of a hollow wire which is provided with a polymer-based inner layer, such as by coating the inside of a metallic hollow wire with a polymer. This provides a smooth surface allowing the sample to slide further into the base member as further samples are acquired. It may be noted that this portion proximal to the endtube formed by coating an inside of a hollow wire may result in a portion having a smooth surface in the sense that it has a low friction but that the Ra-value will be higher than discussed above since the inner surface of the hollow wire, due to the braiding, is not a flat surface. However, the endtube will in such a design be smooth, preferably with an Ra-value as discussed above in order to provide the intended adhesion during the acquiring of the sample. The coating may e.g. be provided by so-called dip coating.

The smooth inner surface is preferably formed of a polymer-based material. The polymer-based material may be of a grade commonly referred to as a non-stick polymer. It is advantageous to use a non-stick grade polymer since this reduces the friction between a first tissue sample and the smooth surface and facilitates the transport of the first tissue sample further into the elongated tubular member. Moreover, surfaces that typically are considered non-stick are often smooth enough to provide the desired smoothness. The polymer-based material may e.g. be ethylene tetrafluoroethylene, TFE. It is also conceivable to use other plastic materials such as other fluoropolymers. Such fluoropolymers may e.g. be polytetrafluorethylene, PTFE, perfluoroalkoxy, PFA, fluorinated ethylene propylene, FEP, and ETFE, ethylene tetrafluoroethylene.

It may be noted that the polymer-based material may be provided in various different physical designs. The polymer-based material may be provided in the form of an elongated tubular member. The polymer-based material may be attached to an inside of an outer member. The polymer-based material may be provided inside an outer member and be movable and rotatable relative to the outer member. The polymer-based material may be provided as a coating inside an outer member. The various physical designs will be discussed in more detail below.

The base member preferably comprises an elongated hollow tubular member extending from the proximal end to the distal end of the base member. Having the base member comprising an elongated hollow tubular member extending all the way from the proximal end to the distal end facilitates e.g. manufacture since the complete length of the base member may be designed in the same manner. Moreover, having the base member comprising an elongated hollow tubular member extending all the way from the proximal end to the distal end facilitates harvesting of the biopsy sample, since it thereby becomes possible to use a mechanical tool, e.g. a flexible metal stylet, extending through the complete biopsy instrument from the proximal end to the distal end such that the samples may securely be pushed out. An elongated hollow tube also allows for harvesting using a burst of air or injecting fluid at the proximal end pushing the samples out at the distal end. These methods would require that the elongated tube is sufficiently air tight or sufficiently liquid tight such that a sufficient amount of the burst of air or liquid actually pushes the samples out. The elongated hollow tube is preferably designed with a uniform cross-section extending from the proximal end to the distal end; apart from that it is provided with localised irregularities in the form of specific design features at the proximal end as such and/or at the distal end as such. These localised irregularities may e.g. be that the hollow tube is at the proximal end provided with a connector and/or that the hollow tube is at the distal end specifically design to provide a cutting edge or specifically designed to receive a separate member providing said cutting edge.

The inner elongated hollow tubular member is preferably formed of a polymer-based material providing said smooth interior surface. This is a convenient manner of providing a smooth interior surface.

The polymer-based material forming the smooth inner surface is preferably provided as a film, preferably a tubular film, which is inserted into the inner elongated hollow tubular member and which is attached to an inside surface of the inner elongated hollow tubular member. The tubular film of polymer-based material may e.g. be attached to the inside surface of the inner elongated hollow tubular member by heating the polymer-based material, directly or indirectly, such that it sticks to the inside surface of the inner elongated hollow tubular member.

Alternatively, the polymer-based material forming the smooth inner surface is provided as a coating.

The inner elongated hollow tubular member preferably comprises a hollow metallic wire rope capable of transferring a force along the central geometrical axis such that a movement of the proximal end along the central geometrical axis is transferred to a movement of the distal end along the central geometrical axis, and of transferring a torque about the central geometrical axis such that a rotation and a torque applied by the motor at the proximal end about the central geometrical axis is transferred from the proximal end to the distal end thereby rotating the distal end about the central geometrical axis.

The inner elongated hollow tubular member is preferably at a distal end thereof provided with said distally facing circular cutting edge.

The outer elongated hollow tubular member comprises preferably also a hollow metallic wire rope.

The inner elongated hollow tubular member is arranged inside the outer elongated hollow tubular member and is rotationally and translationally movable relative to the outer elongated hollow tubular member. One advantage with this design is that the outer elongated hollow tubular member may be kept stationary relative to the endoscope during the sample acquiring process. It is intended that the inner elongated hollow tubular member is to be advanced into the tissue while the outer elongated hollow tubular member remains outside the tissue. By having a distal end of the outer elongated hollow tubular member being positioned outside the tissue and by advancing the distal end of the inner elongated hollow tubular member into the tissue it facilitates having good control on the insertion depth. The fact that the outer elongated hollow tubular member may be kept stationary relative to the endoscope during the sample acquiring process also making it possible to provide the distal end of the outer elongated hollow tubular member with a stopper preventing the distal end from being unintentionally advanced into the tissue. Moreover, by having an outer elongated hollow tubular member which may be kept stationary relative to the endoscope during the sample acquiring process in combination with an inner elongated hollow tubular member being rotationally and translationally movable relative to the outer elongated hollow tubular member the outer elongated hollow tubular member may be designed with a comparably close fit to the working channel of the endoscope. Moreover, since the relative movement is provided between two components of an instrument being specifically designed and manufactured for interaction with each other, it is possible to provide a comparable close fit between the inner and outer elongated hollow tubular members and still secure that sufficient play is provided. Moreover, by being able to use a close fit, the inner and outer elongated hollow tubular members will in a sense support each other and prevent each other from collapsing, which in turn makes it possible to use comparably thin material thicknesses in both the outer and inner elongated hollow tubular members. This will in turn make it possible to have an inner diameter of the distal end of the inner elongated hollow tubular member being comparably large for a given working channel having a given interior diameter. Other advantages and specific design features made by the second embodiment will be discussed in more detail in the detailed description in relation to the drawings.

Preferably, the rotational movability of the inner elongated hollow tubular member is independent from the translational movability such that the inner elongated hollow tubular member may be rotated by a motor and be moved back and forth relative to the outer elongated hollow tubular member independently of the rotational movement.

It may be noted that also in this embodiment - with an inner elongated hollow tubular member being arranged inside the outer elongated hollow tubular member and being rotationally and translationally movable relative to the outer elongated hollow tubular member - the base member may from a bending perspective in accordance with one embodiment be rigid and in accordance with another embodiment be flexible. In the rigid embodiment the base member extends with the central geometrical axis extending along a straight line. Such a rigid biopsy instrument is typically used as a separate biopsy instrument. In such an embodiment the base member may be formed as a needle with a removable inner stylet. The rigid biopsy instrument allows for percutaneous access to a tumour. Typically, in such an embodiment the outer elongated hollow tubular member is fixed, and an inner elongated hollow tubular member is rotated by the motorized handle and advanced into tissue after the stylet has been withdrawn. Once the rigid inner stylet has been fully removed the inner hollow tube may be drilled into a hollow space like the abdomen, chest, sinus or joint and used to insert other instruments like cameras, injection devices for fluid or gas or guidewires/rods. In accordance with another embodiment of the embodiment with an inner elongated hollow tubular member being arranged inside the outer elongated hollow tubular member and being rotationally and translationally movable relative to the outer elongated hollow tubular member, the base member is from a bending perspective flexible whereby it is capable of extending along a central geometrical axis having various shapes, which is typically required for a biopsy instrument for use in an endoscope. Such a flexible biopsy instrument for use in an endoscope is sometimes referred to as an endoscopic biopsy instrument.

The flexible inner tube may be used to insert a flexible guide wire and then removed with guide wire in position to be used for insertion of other instruments like stents and dilatation balloons.

The inner elongated hollow tubular member is preferably capable of transferring a force along the central geometrical axis such that a movement of the proximal end along the central geometrical axis is transferred to a movement of the distal end along the central geometrical axis, and of transferring a torque about the central geometrical axis such that a rotation and a torque applied by a motor at the proximal end about the central geometrical axis is transferred from the proximal end to the distal end thereby rotating the distal end about the central geometrical axis.

The inner elongated hollow tubular member has preferably at a proximal end thereof a connector for connection, preferably a releasable connection, to a motor, the connector being capable of transferring said movement along the central geometrical axis and said rotation and torque.

The above object has also been achieved by a biopsy instrument comprising
an outer elongated hollow tubular member which extends from a proximal end to a distal end along a central geometrical axis, and
a base member which extends from a proximal end to a distal end along the central geometrical axis, wherein at least a distal end portion of the base member is shaped as an elongated hollow tube, the elongated hollow tube at the distal end of the base member being intended to be at least partly inserted into a tissue from which a biopsy is to be obtained,
wherein the base member is arranged inside the outer elongated hollow tubular member and is independently rotationally and translationally movable relative to the outer elongated hollow tubular member,
wherein the base member is capable of transferring a force along the central geometrical axis such that a movement of the proximal end of the base member along the central geometrical axis is transferred to a movement of the distal end of the base member along the central geometrical axis, and of transferring a torque about the central geometrical axis such that a rotation and a torque applied by a motor at the proximal end of the base member about the central geometrical axis is transferred from the proximal end of the base member to the distal end of the base member thereby rotating the distal end of the base member about the central geometrical axis,
wherein the elongated hollow tube is capable of being advanced out of a distal end of the outer elongated hollow tubular member and to be retracted back into the outer elongated hollow tubular member by a movement of the proximal end of the base member along the central geometrical axis, while being rotated inside and relative to the outer elongated hollow tubular member about the central geometrical axis by the motor applying a rotation and a torque at the proximal end of the base member,
wherein the elongated hollow tube is provided with a distally facing circular cutting edge defining a mouth of the distal end of the elongated hollow tube,
wherein the elongated hollow tube has, at a distal portion of the elongated hollow tube, a hollow elongated tubular sample acquiring portion having a smooth interior surface,
wherein the proximal end of the base member is configured to be connected to the motor such that rotation and torque may be applied by the motor to the proximal end of the base member and be transferred by the base member to the elongated hollow tube at the distal end of the base member,
wherein the motor is configured to provide a rotation of the elongated hollow tube, while the elongated hollow tube is advanced out of the distal end of the outer elongated hollow tubular member and is retracted back into the outer elongated hollow tubular member, inside and relative to the outer elongated hollow tubular member about the central geometrical axis by applying rotation and torque to the proximal end of the base member,
wherein the smooth inner surface has a surface roughness with an Ra value of less than 1,5µm, preferably less than 1 µm, when formed of steel, such as a medical grade stainless steel, and less than 6 µm, such as between 1 µm and 6 µm, when formed of a polymer-based material.

The above object has also been achieved by a method of acquiring a biopsy, the method comprising:
providing a biopsy instrument comprising
   an outer elongated hollow tubular member which extends from a proximal end to a distal end along a central geometrical axis, and
   a base member which extends from a proximal end to a distal end along the central geometrical axis, wherein at least a distal end portion of the base member is shaped as an elongated hollow tube elongated hollow tube having a distally facing circular cutting edge defining a mouth of the distal end of the hollow tube at the distal end being intended to be at least partly inserted into a tissue from which a biopsy is to be obtained,
   wherein the base member is arranged inside the outer elongated hollow tubular member and is independently rotationally and translationally movable relative to the outer elongated hollow tubular member,
providing a manoeuvring unit having a motor,
connecting a proximal end of the base member to the motor,
connecting a proximal end of the outer elongated hollow tubular member to the manoeuvring unit,
moving a distal end of the biopsy instrument to a position where a tissue sample is to be acquired, preferably with the distal end of the base member being positioned inside the outer elongated hollow tubular member, activating the motor such that rotation at a rotational speed of at least 13 000 rpm is transferred to the distal end of the biopsy instrument,
advancing the elongated hollow tube with the distally facing circular cutting edge into the tissue from which a tissue sample is to be obtained, while the distal end is being rotated by the motor at a rotational speed of at least 13 000 rpm thereby cutting a core of the tissue which, due to the advancement of the elongated hollow tube, enters relative to the elongated hollow tube through the mouth into a sample acquiring portion of the elongated hollow tube,
retracting the distal end of the base member out of the tissue while the distal end of the base member is being rotated by the motor with a circumferential outer surface of the core at least partly abutting a smooth interior surface of a hollow elongated tubular sample acquiring portion being provided at a distal portion of the elongated hollow tube,
whereby the core of the tissue is detached from the tissue by a pulling force due to the retraction of the elongated hollow tube and due to an adhesive force formed at an interface between the smooth interior surface and the circumferential outer surface of the core which force keeps the core inside the sample acquiring portion having the smooth interior surface.

The above object has also been achieved by a kit of parts comprising
a biopsy instrument of the kind disclosed in its basic configuration or in any of the preferred embodiments, and
a manoeuvring unit comprising a motor,
wherein the biopsy instrument is at its proximal end connectable to the motor such that rotation and torque may be applied by the motor to the proximal end of the base member and transferred to the distal end of the base member.

The above object has also been achieved by a method of acquiring a biopsy, the method comprising:
connecting a proximal end of a biopsy instrument to a manoeuvring unit having a motor,
moving a distal end of the biopsy instrument to a position where a tissue sample is to be acquired,
activating the motor such that rotation is transferred to the distal end of the biopsy instrument,
advancing the distal end, which at at least a distal end portion of the base member is shaped as an elongated hollow tube having a distally facing circular cutting edge defining a mouth of the distal end of the hollow tube, into the tissue from which a tissue sample is to be obtained while the distal end is being rotated by the motor thereby cutting a core of the tissue which, due to the advancement of the hollow tube, enters relative to the hollow tube through the mouth into a sample acquiring portion of the hollow tube,
retracting the distal end out of the tissue while the distal end is being rotated by the motor with a circumferential outer surface of the core at least partly abutting a smooth interior surface of a hollow elongated tubular sample acquiring portion being provided at a distal portion of the hollow tube,
whereby the core of the tissue is detached from the tissue by a pulling force due to the retraction of the hollow tube and due to an adhesive force formed at an interface between the smooth interior surface and the circumferential outer surface of the core which force keeps the core inside the sample acquiring portion having the smooth interior surface.

The above object has also been achieved by a biopsy instrument comprising
a base member which extends from a proximal end to a distal end along a central geometrical axis, wherein at least a distal end portion of the base member is shaped as an elongated hollow tube, the distal end being intended to be at least partly inserted into a tissue from which a biopsy is to be obtained,
wherein the base member may be capable of transferring a force along the central geometrical axis such that a movement of the proximal end along the central geometrical axis is transferred to a movement of the distal end along the central geometrical axis, and of transferring a torque about the central geometrical axis such that a rotation and a torque applied by a motor at the proximal end about the central geometrical axis is transferred from the proximal end to the distal end thereby rotating the distal end about the central geometrical axis,
wherein the hollow tube may be provided with a distally facing circular cutting edge defining a mouth of the distal end of the hollow tube,
wherein the hollow tube may have, at a distal portion of the hollow tube, a hollow elongated tubular sample acquiring portion having a smooth interior surface,
wherein the smooth interior surface is preferably smooth to such an extent that when a reference biopsy is to be acquired, the cutting edge and the distal end of the hollow tube is configured to be advanced along the central geometrical axis into a tissue while being rotated by being motor driven at its proximal end and thereby cutting a core of the tissue which, due to the advancement of the hollow tube, enters relative to the hollow tube through the mouth into the sample acquiring portion of the hollow tube with a circumferential outer surface of the core at least partly abutting the smooth interior surface of the sample acquiring portion, where-after the hollow tube is retracted from the tissue while being rotated by being motor driven at its proximal end whereby the core of the tissue is detached from the tissue by a pulling force due to the retraction of the hollow tube and due to an adhesive force formed at an interface between the smooth interior surface and the circumferential outer surface of the core which force keeps the core inside the sample acquiring portion having the smooth interior surface. The adhesive force in combination with rotation produces a rotation of the sample at the most distal end when the sample is pulled back, in effect releasing it from the tissue by the increasingly thinning and twisted thread produced by the rotation of the biopsy.

The above object has also been achieved by a biopsy instrument comprising a base member which extends from a proximal end to a distal end along a central geometrical axis, wherein at least a distal end portion of the base member is shaped as an elongated hollow tube, the distal end being intended to be at least partly inserted into a tissue from which a biopsy is to be obtained, wherein the hollow tube is provided with a distally facing circular cutting edge defining a mouth of the distal end of the hollow tube, wherein the hollow tube has, at a distal portion of the hollow tube, a hollow elongated tubular sample acquiring portion having a smooth interior surface.

It may be noted that it is also conceivable that for some user scenarios, the inner elongated hollow tubular member (rigid or flexible) can be rotated manually at the proximal end resulting in a distally facing circular cutting edge.

### Brief description of the drawings

The invention will by way of example be described in more detail with reference to the appended schematic drawings, which shows a presently preferred embodiment of the invention.
Figure 1a discloses schematically a physician using a biopsy instrument according to one embodiment and an endoscope to obtain a tissue sample from a patient.
Figure 1b discloses schematically a physician using a biopsy instrument according to another embodiment and an endoscope to obtain a tissue sample from a patient
Figure 2a discloses in more detail the proximal end of the endoscope and the proximal end of the biopsy instrument of figure 1a.
Figure 2b discloses in more detail the proximal end of the endoscope and the proximal end of the biopsy instrument of figure 1b.
Figure 3a discloses the distal end of the outer sheath and the distal end of the biopsy instrument being advanced into the tissue from which a sample is to be acquired.
Figure 3a discloses the distal end of the endoscope and the distal end of base member, the distal end of base member being advanced out of an outer elongated hollow member and into the tissue from which a sample is to be acquired.
Figure 3b discloses the distal end of the endoscope and the distal end of the base member being advanced into the tissue from which a sample is to be acquired.
Figure 4 discloses the endoscope and instrument shown in figure 3b after several samples has been acquired.

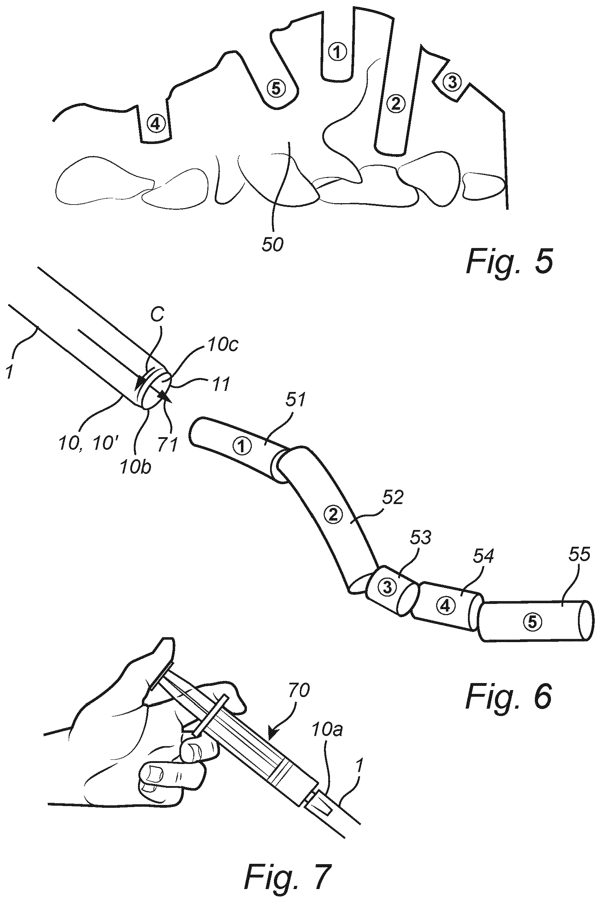

Figure 5 shows the tissue after several samples has been acquired.
Figure 6 discloses harvesting of the samples from the biopsy instrument.
Figure 7 discloses harvesting using an overpressure provided by a syringe.

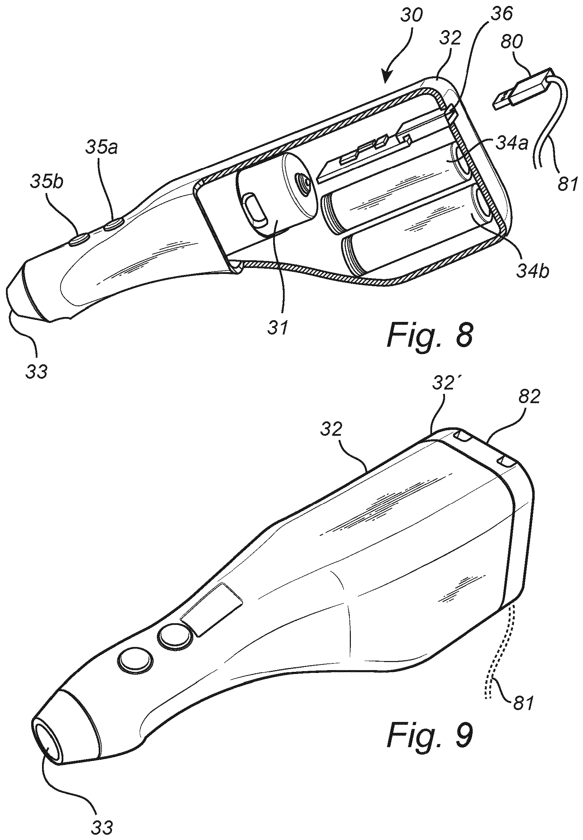

Figure 8 discloses an inside of a manoeuvring member configured to be attached to the proximal end of the biopsy instrument.
Figure 9 discloses the outside and manoeuvring buttons of the manoeuvring member of figure 8.

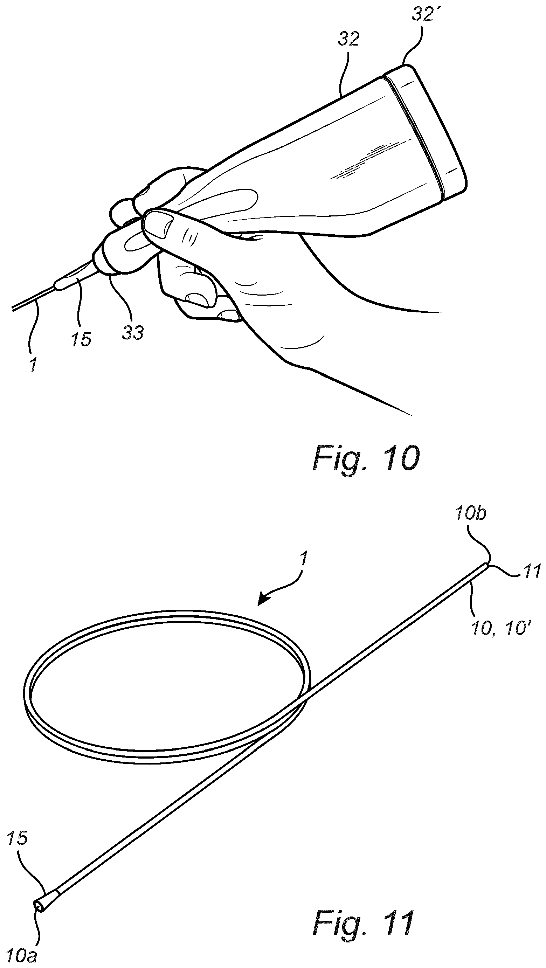

Figure 10 discloses the manoeuvring member being attached to the proximal end of the biopsy instrument.
Figure 11 discloses a flexible biopsy instrument.

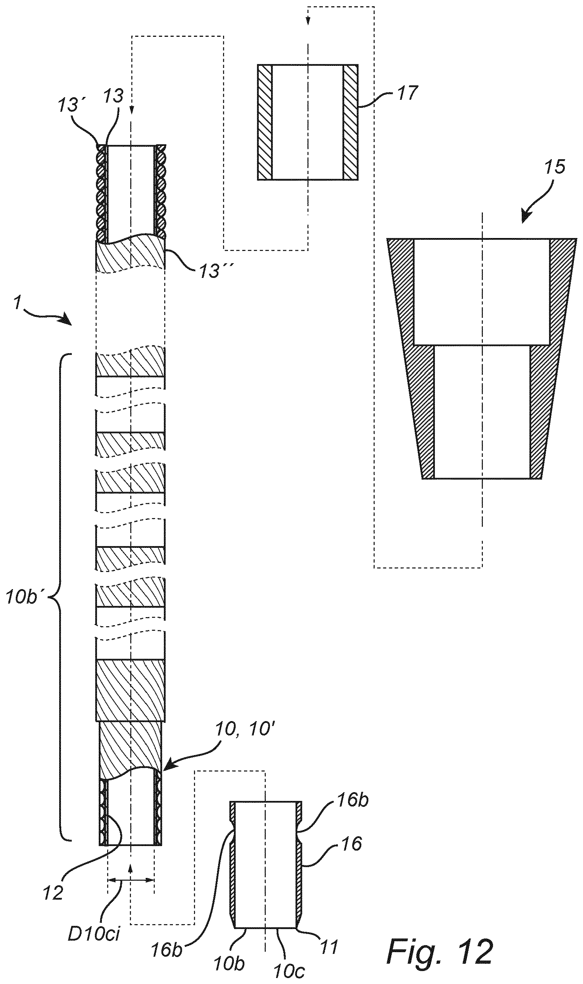

Figure 12 discloses an inner elongated flexible hollow member in more detail in a cross-sectional and exploded view.
Figure 13a-b discloses a first and a second position of the distal end of a biopsy instrument while acquiring a tissue sample along a surface of the tissue.

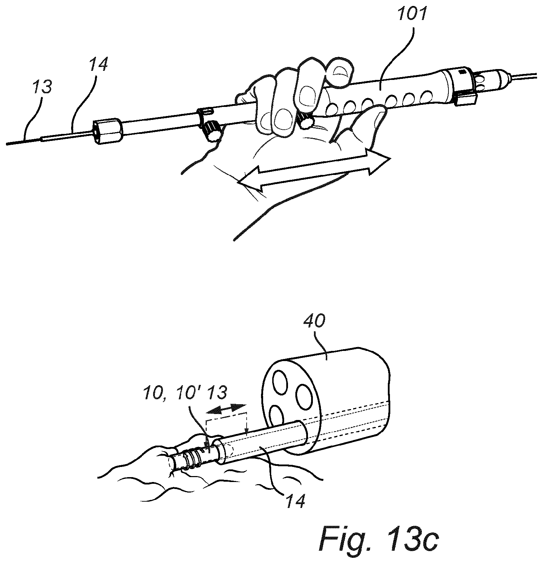

Figure 13c discloses how a telescopic function may be manoeuvred to a acquire tissue sample along a surface of the tissue.
Figure 14a discloses the distal end of the biopsy instrument while acquiring a tissue sample along a surface of the tissue as seen in a cross-sectional view of figures 13a-b.
Figure 14b discloses the tissue having a groove in the surface being formed by the biopsy instrument as shown in figures 13a-b and 14a.
Figure 14c is a top-plan view of the tissue and the groove of figure 14b.

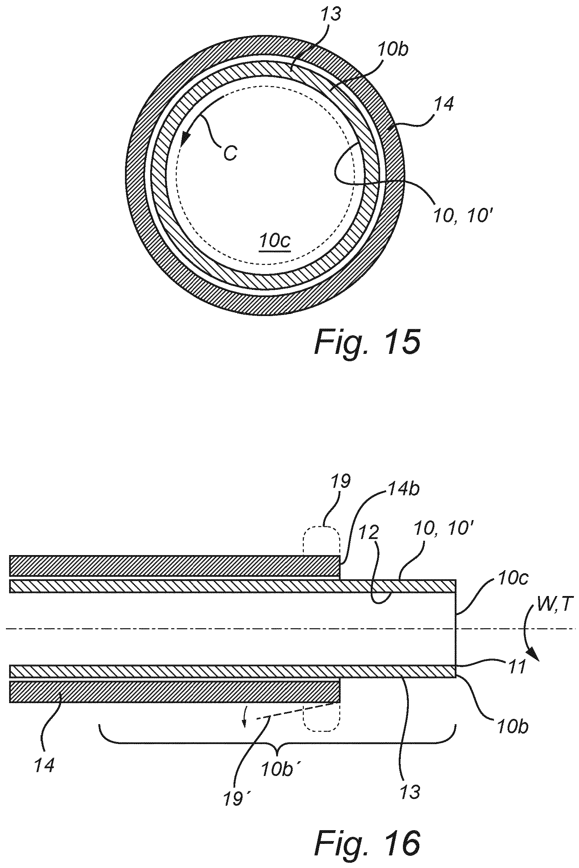

Figure 15 is a cross-sectional view of a biopsy instrument in accordance with a second embodiment.
Figure 16 is another cross-sectional view of the biopsy instrument of figure 15.

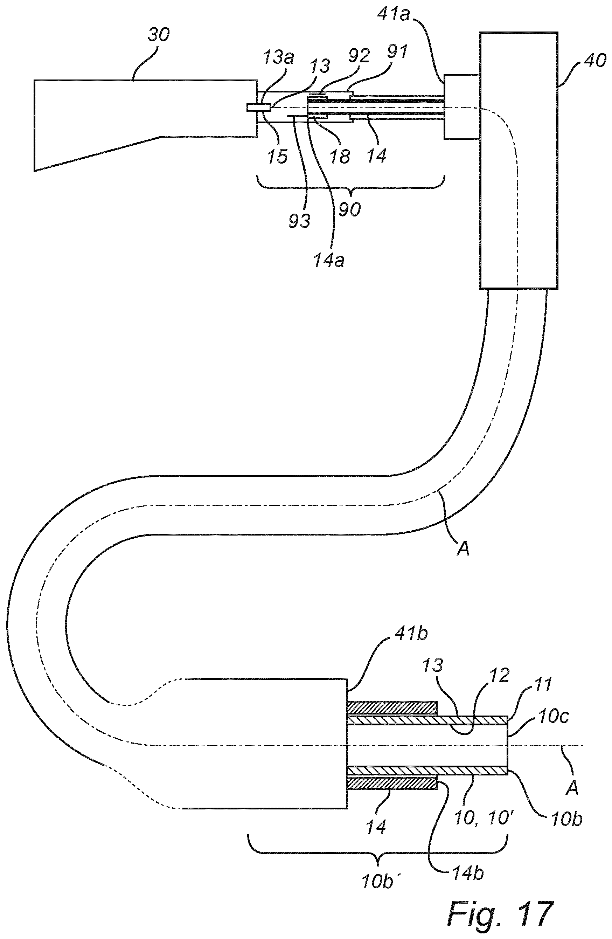

Figure 17 is a schematic view disclosing the biopsy instrument of figures 15 and 16 in use in an endoscope.

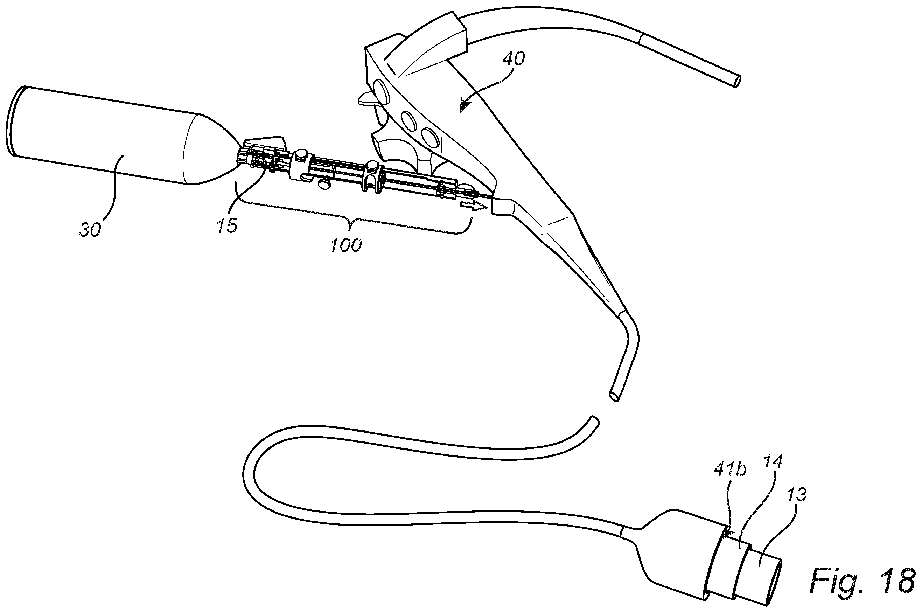

Figure 18 is a schematic view disclosing a biopsy instrument of the same kind as in figures 15-17 being connected to a variant of a telescope mechanism between the motor and the biopsy instrument.

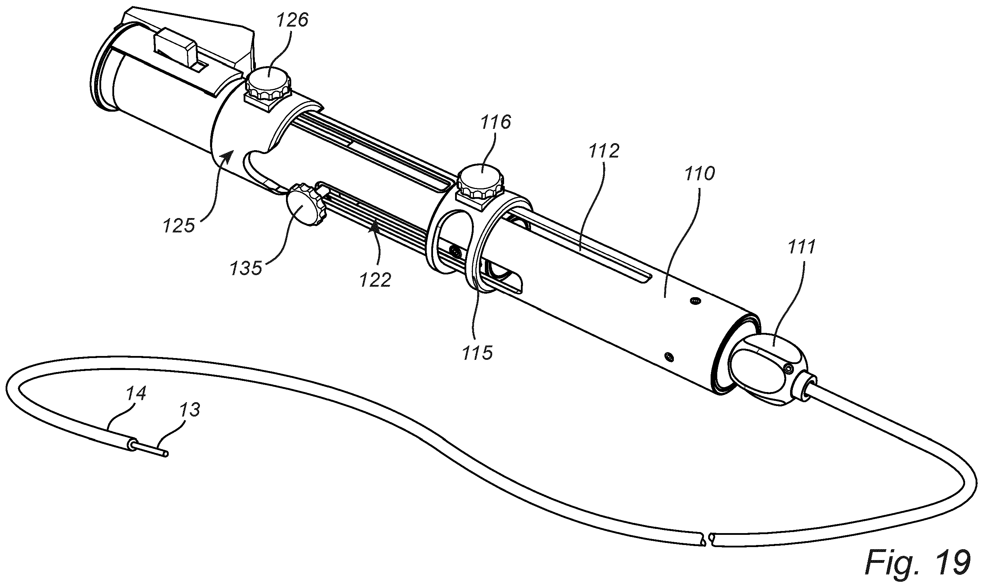

Figure 19 discloses in more detail the telescope mechanism shown in figure 18.

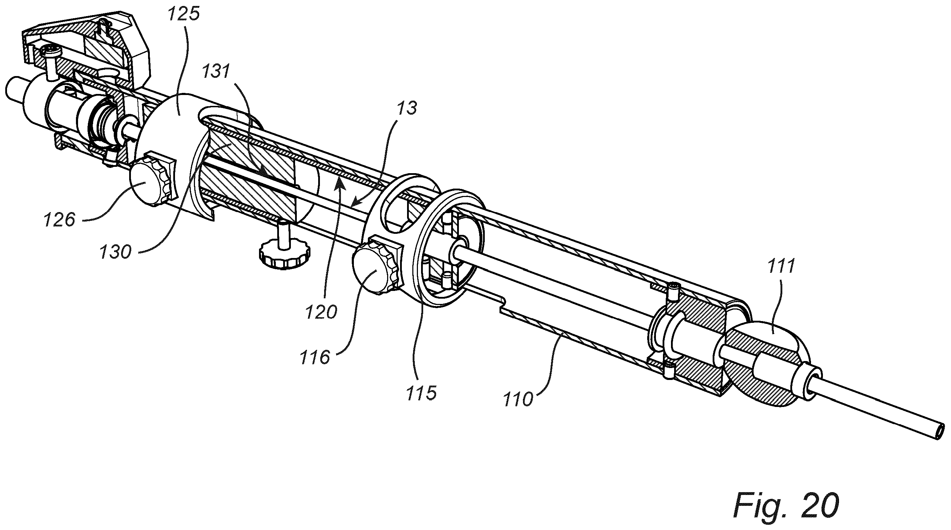

Figure 20 is a cross-sectional view of the telescope mechanism of figure 18 and 19.

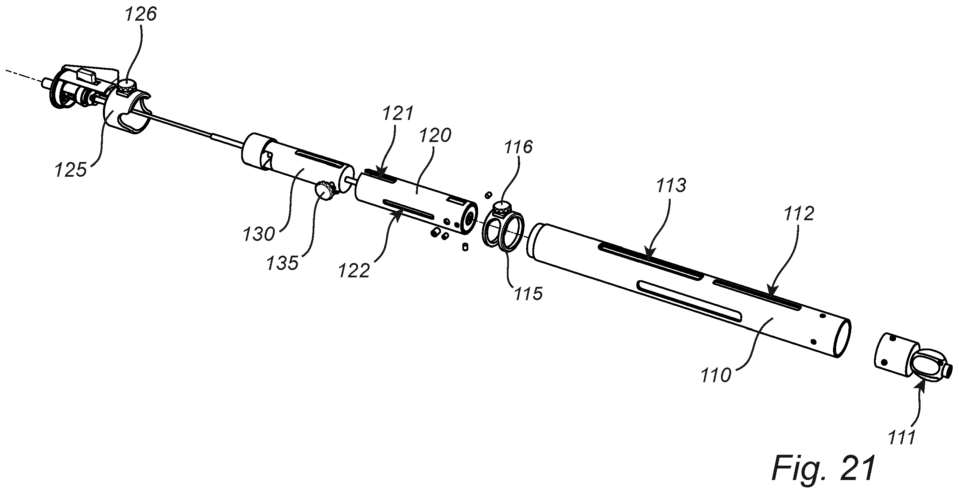

Figure 21 is an exploded view of the telescope mechanism of figures 18-20.

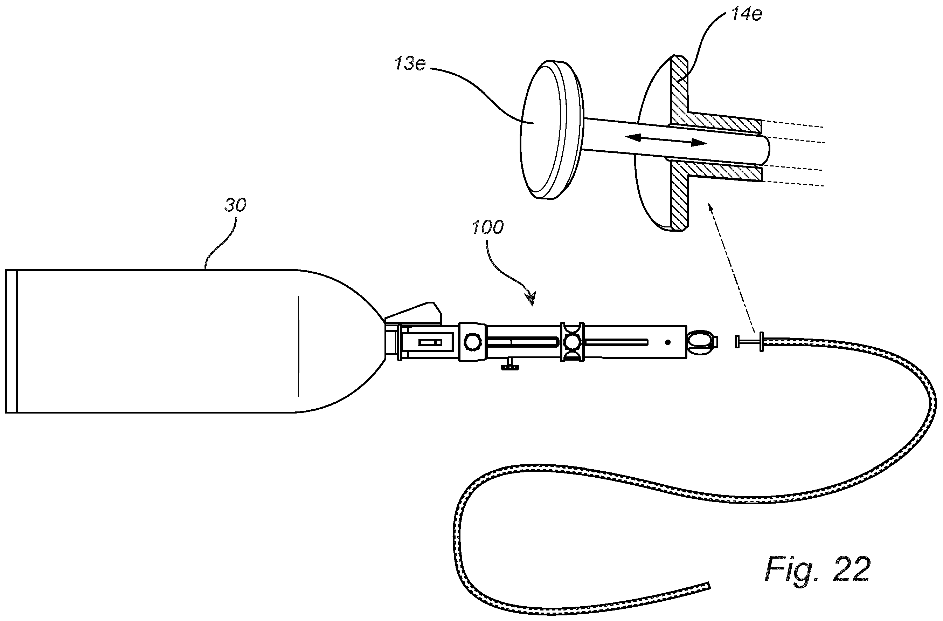

Figure 22 discloses a motor, a telescope mechanism and a biopsy instrument and discloses schematically an example of an interface of the biopsy instrument being configured to be connected to the telescope mechanism.
Figure 23 discloses an outer rigid hollow needle and an inner rigid hollow needle configured to be positioned inside the outer rigid hollow needle.
Figure 24 discloses the inner rigid hollow needle being inserted into the rigid outer hollow needle.

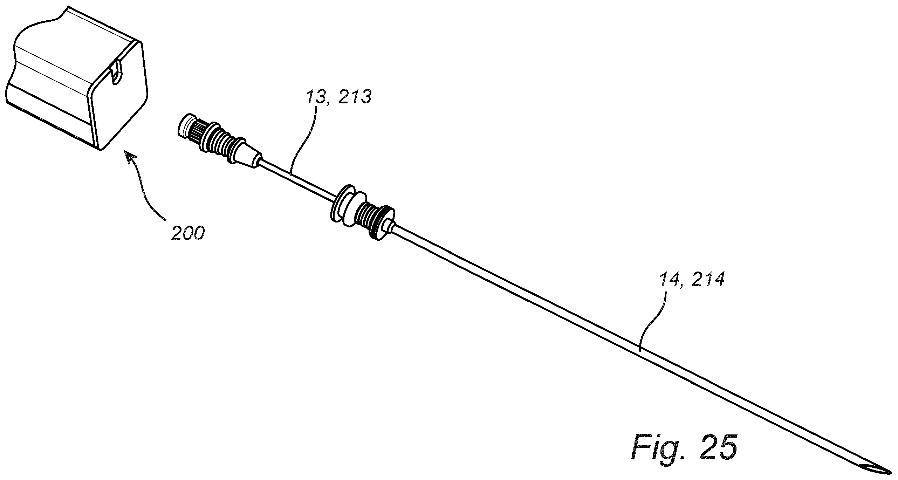

Figure 25 discloses the inner rigid hollow needle and the outer rigid hollow needle in a retracted position of the inner rigid hollow needle in which position the needles are configured to be handled and to be inserted into a handle for operation in the sample acquiring method.

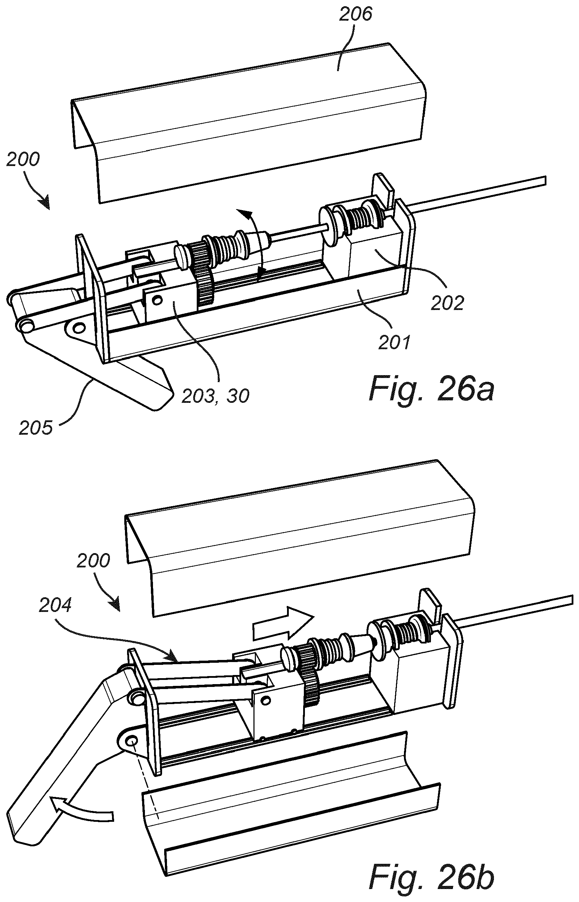

Figure 26a discloses the needles positioned in the handle and in a state ready to acquire a biopsy sample.
Figure 26b discloses schematically operation of the handle to acquire a biopsy sample.

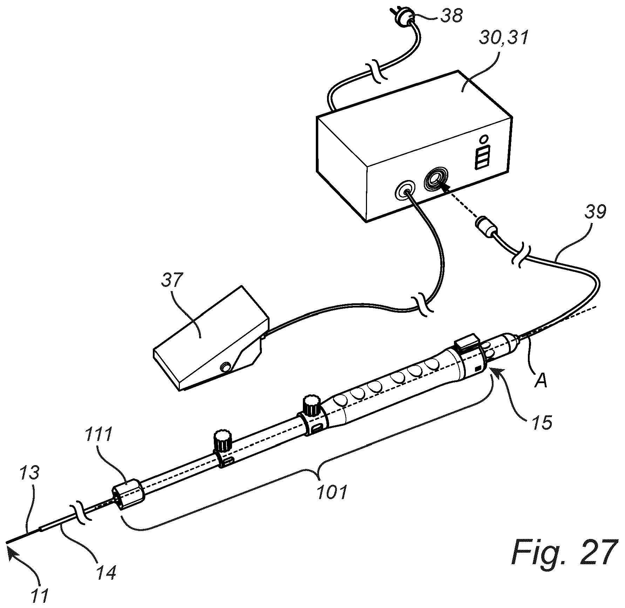

Figure 27 is an overview of a system comprising a motor, a telescope mechanism and a biopsy instrument.

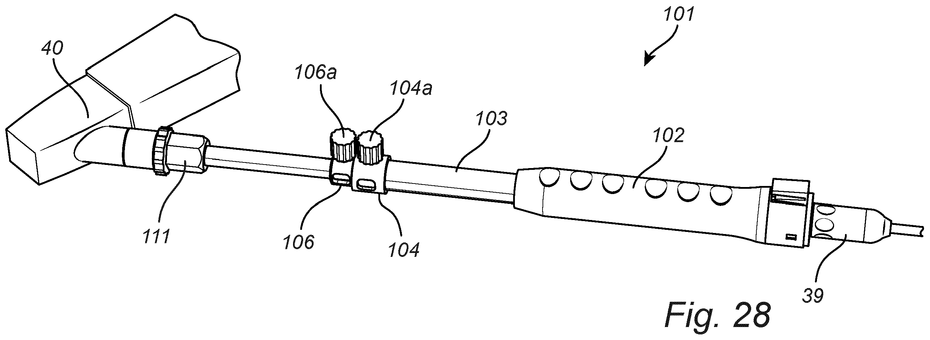

Figure 28 shows the telescope mechanism of figure 27 connected to an endoscope.

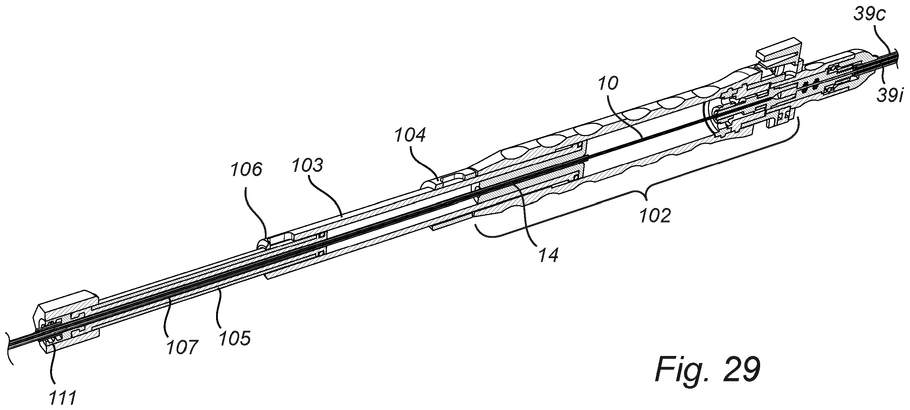

Figures 29-30 in more detail the telescope mechanism shown in figures 27-28.

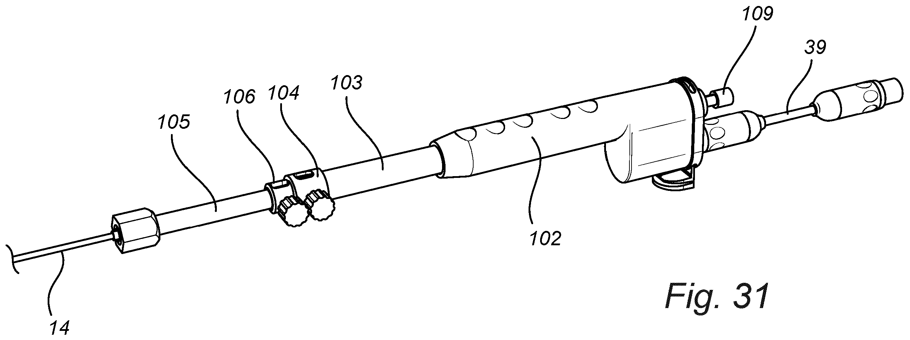

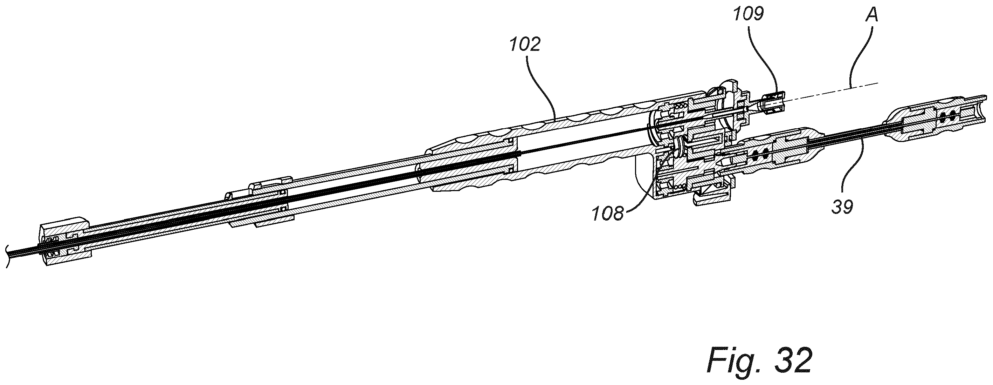

Figures 31-32 discloses a variant of the telescope mechanism shown in figures 27-30.

### Detailed description of preferred embodiments

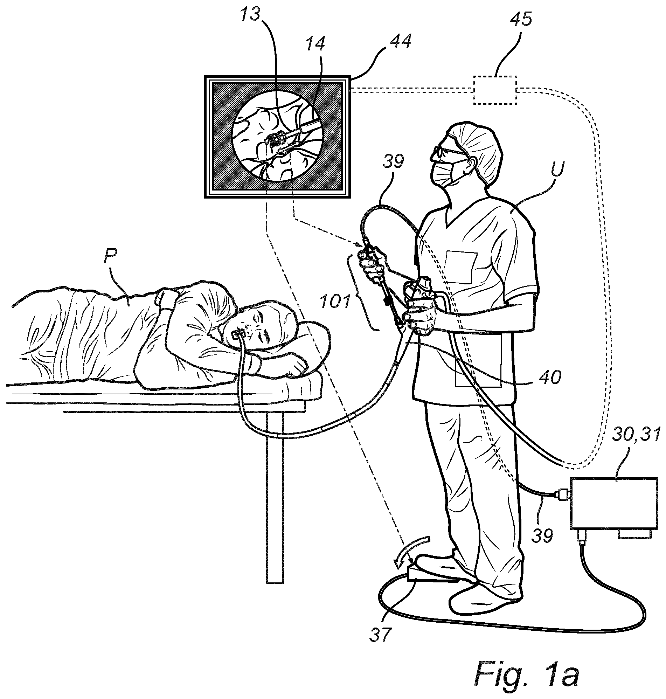

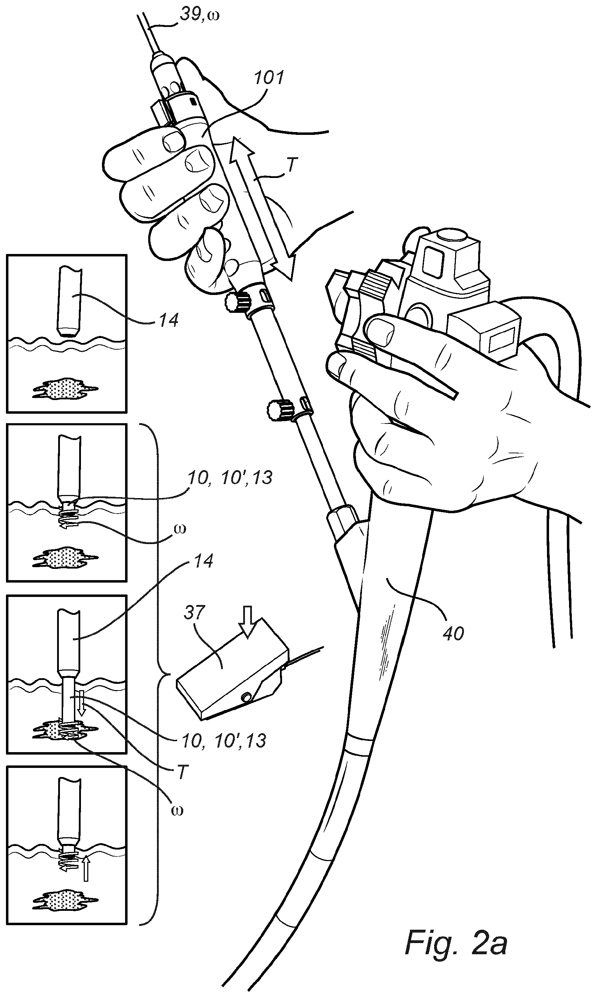

In figures 1a-b, there is generally disclosed how a user U, such as a physician, uses an endoscope 40 to guide a biopsy instrument 1 to a sample site 50 through a body cavity of a patient P. The biopsy instrument 1 is inserted into the patient's body to the intended sample site 50 by the endoscope 40 being inserted through a body cavity of the patient and with the biopsy instrument 1 being inserted in a working channel 41 of the endoscope 40. As shown in figures 1a-b and in more detail in figures 2a-b, the endoscope is provided with an access opening 41a at the proximal end of the remaining outside of the patient's body, wherein the biopsy instrument 1 is intended to be inserted into the endoscope via the access opening 41a. The endoscope 40 is typically provided with a camera and/or an ultrasound probe and is typically connected to a screen 44 via a processing unit 45 capable of transform the data from the camera or ultrasound probe into an image on the screen 44.

The biopsy instrument 1 comprises a base member 10 which extends from a proximal end 10a to a distal end 10b along a central geometrical axis A.

One embodiment of the complete biopsy instrument 1 is shown in figure 11. In the embodiment shown in figure 11, the base member 10 is from a bending perspective flexible. It is thereby capable of extending along a central geometrical axis A having various shapes, which is typically required for a biopsy instrument 1 for use in an endoscope 40. Such a flexible biopsy instrument 1 for use in an endoscope 40 is sometimes referred to as an endoscopic biopsy instrument 1. However, it may be noted that the biopsy instrument 1 is also useful for applications where it is not used in an endoscope. In such an instance it may, from a bending perspective, be rigid and extend with the central geometrical axis A extending along a straight line. Such a rigid biopsy instrument is typically used as a separate biopsy instrument 1.

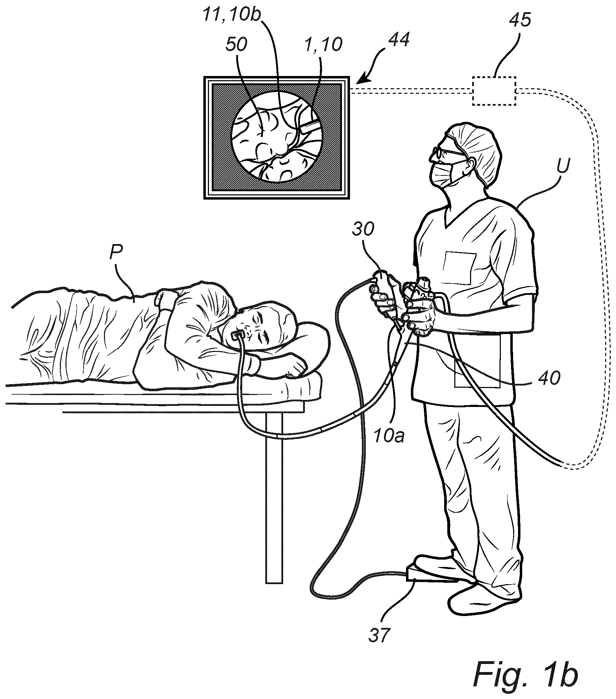

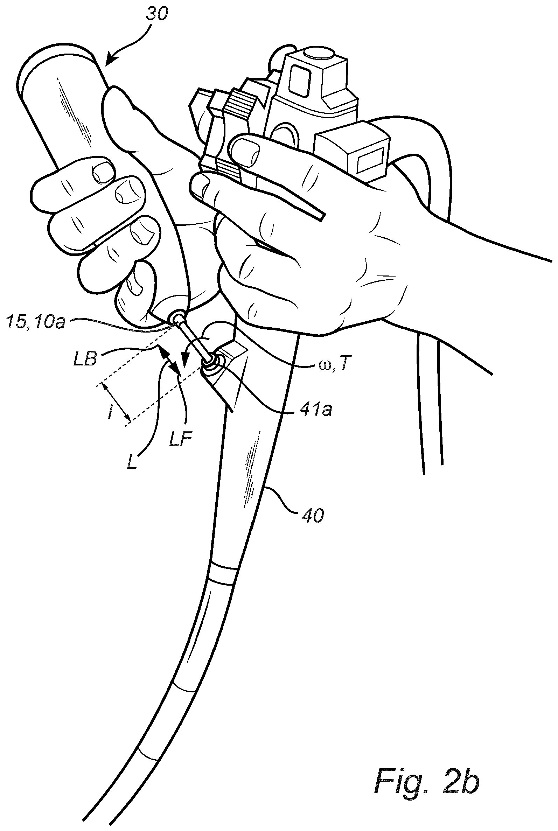

The proximal end 10a is shown in its context in figures 1 and 2 and the distal end 10b is shown in its context e.g. in figures 3 and 4.

It may be noted that in figure 1a, the manoeuvring unit 30 with the motor 31 is a separate box configured to be positioned on a shelf or the like. The biopsy instrument comprises a telescopic mechanism 101 and is connected to the motor 31 via a drive wire 39. More details are provided with reference e.g. to figures 27-32.

It may be noted that in figure 1b, the manoeuvring unit 30 with the motor 31 is designed as a hand-held part.

It may be noted that in the description related to the manoeuvring of the distal end of the biopsy instrument, it is in most cases conceivable to use either one of the manoeuvring units 30 of figure 1a or figure 1b.

As is shown e.g. in figures 3a-b and 4, at least a distal end portion 10b' of the base member 10 is shaped as an elongated hollow tube 10'. In the preferred embodiments shown in detail in figure 12 and 15-16, respectively, the base member 10 is shaped as a hollow tube 10' extending from the proximal end 10a to the distal end 10b of the base member 10.

As is shown in figures 3a-b and 4, the distal end 10b, being shaped as an elongated hollow tube 10', is intended to be at least partly inserted into a tissue 50 from which a biopsy is to be obtained. In the user case shown in figures 3a-b and 4, the distal end 10b is fully inserted into the tissue in the sense that the distal end 10b is inserted with the complete circumference C inserted into the tissue 50. In the user case shown in figures 13a-b and 14a-c, the distal end 10b is only partly inserted into the tissue in the sense that the distal end 10b is inserted with only a portion of the complete circumference C being inserted into the tissue 50.

The base member 10 is capable of transferring a force along the central geometrical axis A such that a movement LF, LB of the proximal end 10a along the central geometrical axis A is transferred to a movement LF, LB of the distal end 10b along the central geometrical axis A. The base member 10 is also capable of transferring a torque about the central geometrical axis A such that a rotation ω and a torque T applied by a motor 31 at the proximal end 10a about the central geometrical axis A is transferred from the proximal end 10a to the distal end 10b thereby rotating the distal end 10b about the central geometrical axis A. The distal end 10b of the base member 10 is thereby manoeuvrable by advancing and retracting the proximal end 10a and by applying a rotation ω and a torque T at the proximal end 10a.

The biopsy instrument 1 is intended to be used in accordance with the brief disclosure presented above with reference to figures 1a-b. The intended method of use will in the following be disclosed in more detail with reference to figures 1a-b and 2a-b. The user U has connected a proximal end 10a of a biopsy instrument 1 to a manoeuvring unit 30 having a motor 31. By moving the endoscope 40 and then by moving a distal end 10b of the biopsy instrument 1 relative to the endoscope 40, the distal end 10b of the biopsy instrument 1 is moved to a position where a tissue sample is to be acquired. The user U is in this movement guided by the image on the screen 44. Thereafter, the user U activates the motor 31 such that rotation is transferred to the distal end 10b of the biopsy instrument 1. Thereafter, the user U advances the distal end 10b, which at at least a distal end portion 10b' of the base member 10 is shaped as an elongated hollow tube 10' having a distally facing circular cutting edge 11 defining a mouth 10c of the distal end 10b of the hollow tube 10', into the tissue 50 from which a tissue sample is to be obtained while the distal end 10b is being rotated by the motor 31 thereby cutting a core 51 of the tissue 50 which, due to the advancement LF of the hollow tube 10', enters relative to the hollow tube 10' through the mouth 10c into a sample acquiring portion 10b' of the hollow tube 10'. This advancement may be said to be that the biopsy instrument 1 is moved relative to the endoscope 40 in a direction extending from the proximal end 10a to the distal end 10b.

This advancement is, in the embodiment of figure 2a, performed by manoeuvring the telescopic mechanism 101. As indicated in the four small figures in figure 2a, the telescopic mechanism 101 is manoeuvred such that an outer elongated hollow tubular member 14, which initially preferably is positioned inside the working channel 41 of the endoscope 40, is moved to a desired position relative to the tissue. Thereafter, the motor 31 is activated such that the inner elongated hollow tube 10' starts to rotate. Thereafter, the telescopic mechanism 101 is manoeuvred such that the base member with the inner elongated hollow tube 10' is advanced out of the outer elongated hollow tubular member 14 and into the tissue. Thereafter the telescopic mechanism 101 is manoeuvred such that the base member with the inner elongated hollow tube 10' is retracted partly of fully into the outer elongated hollow tubular member 14. The advancement and retraction of the inner elongated hollow tube 10' may be repeated until the desired number of samples has been retrieved.

This advancement is, in the embodiment of figure 2b, performed by moving the manoeuvring unit 30 forward along the arrow LF relative to the endoscope 40 and the access opening 41a, such that the free distance I of the biopsy instrument 1 decreases. Once the distal end 10b has been inserted into the tissue 50 to the intended depth d, the user U thereafter retracts the distal end 10b out of the tissue 50 while the distal end 10b is being rotated by the motor 31 with a circumferential outer surface of the core 51 at least partly abutting a smooth interior surface 12 of a hollow elongated tubular sample acquiring portion 10b' being provided at a distal portion 10b' of the hollow tube 10',

The core 51 of the tissue 50 is detached from the tissue 50 by a pulling force due to the retraction LB of the hollow tube 10' and due to an adhesive force formed at an interface between the smooth interior surface 12 and the circumferential outer surface of the core 51 which force keeps the core 51 inside the sample acquiring portion 10b' having the smooth interior surface 12.

In addition, the core 51 may be separated from the tissue 50 by shearing and/or tensile forces. Without being bound by the explanation below, it is believed that the sample acquiring portion rotates with a high rotational speed relative to the tissue whereby a liquid film is formed between the inner surface of the sample acquiring portion and the tissue core, which reduces the friction between the tissue core and the sample acquiring portion. The film formation is enhanced by a high rotational speed.

Likewise, a liquid film may be formed at the outer surface of the sample acquiring portion. The formation of a liquid film is enhanced if the inner surface is smooth, for example having a surface roughness of below 0.5 micrometres. The tissue core is non-rotating as long as the sample acquiring portion is pushed further inside the tissue. When the sample acquiring portion is no longer pushed into the tissue but is retracted, the tissue core inside the sample acquiring portion will adhere to the inner surface of the sample acquiring portion and start to rotate, thereby separating the sample core from the surrounding tissue by shearing forces and tearing or pulling forces. The sample core will now rotate together with the sample acquiring portion. When the next sample core is to be obtained, the previous sample core will be pushed further into the sample acquiring portion against the frictional forces exerted by the inner surface. The friction coefficient should be as small as possible, such as below 0.10 or below 0.06.

The smooth inner surface has preferably a surface roughness with an Ra value of less than 1,5µm, preferably less than 1 µm, when formed of steel, such as a medical grade stainless steel, and less than 6 µm, such as between 1 µm and 6 µm, when formed of a polymer-based material.

As is e.g. schematically shown in figures 3a-b and 4 and is shown in more detail in figures 12 and 16, the hollow tube 10' is provided with a distally facing circular cutting edge 11 defining a mouth 10c of the distal end 10b of the hollow tube 10'. In all the preferred embodiments, both of the flexible variants and for the rigid variants, the distally facing circular cutting edge 11 has, as seen along the circumference C of the mouth 11c a straight-line configuration. It is also preferred that the mouth 11c defines a plane having a normal parallel to the extension of the central geometrical axis A as the central geometrical axis passes through said plane of the mouth 11c. That is, the hollow tube 10' is in an embodiment cut by a plane orthogonal to the longitudinal extension of the hollow tube 10' at the mouth 11c.

The hollow tube 10' has, at a distal portion 10b' of the hollow tube 10', a hollow elongated tubular sample acquiring portion 10b' having a smooth interior surface 12. The tubular sample acquiring portion 10b' has a length along the central geometrical axis A, the length preferably being sufficient to allow a plurality of samples 51, 52, 53, 54, 55 to be collected and positioned one after the other in the tubular sample acquiring portion 10b' along the central geometrical axis A. The length is preferably at least 10 times, and more preferably at least 20 times, the inner diameter D11ci of the hollow tube 10'. However, as mentioned above, the base member 10 is preferably formed of the elongated hollow tube 10' extending from the proximal end 10a to the distal end 10b of the base member 10. Thereby it may be said that the hollow elongated tubular sample acquiring portion 10b' is basically formed all the way from the distal end 10b to the proximal end 10a.

The elongated hollow tube 10' may be designed with a uniform cross-section extending from the proximal end 10a to the distal end 10b; apart from that it is provided with localised irregularities in the form of specific design features at the proximal end 10a as such and/or at the distal end 10b as such. These localised irregularities may e.g. be that the hollow tube 10' is at the proximal end 10a provided with a connector 15 and/or that the hollow tube 10' is at the distal end 10b specifically design to provide a cutting edge 11 or specifically designed to receive a separate member providing said cutting edge 11.

The smooth interior surface 12 is smooth to such an extent that when a reference biopsy is to be acquired in accordance with the method shown in figures 3a-b and 4, the cutting edge 11 and the distal end 10b of the hollow tube 10' is configured to be advanced along the central geometrical axis A into a tissue 50 while being rotated ω, T by being motor driven at its proximal end 10a and thereby cutting a core 51 of the tissue 50 which, due to the advancement LF of the hollow tube 10', enters relative to the hollow tube 10' through the mouth 10c into the sample acquiring portion 10b' of the hollow tube 10' with a circumferential outer surface of the core 51 at least partly abutting the smooth interior surface 12 of the sample acquiring portion 10b', where-after the hollow tube 10' is retracted from the tissue 50 while being rotated ω, T by being motor driven at its proximal end 10a whereby the core 51 of the tissue 50 is detached from the tissue 50 by a pulling force due to the retraction LB of the hollow tube 10' and due to an adhesive force formed at an interface between the smooth interior surface 12 and the circumferential outer surface of the core 51 which force keeps the core 51 inside the sample acquiring portion 10b' having the smooth interior surface 12. The surface 12 is preferably smooth to such an extent that when performing a reference sample with a biopsy instrument 1 of the above kind, a core 51 is, during retraction of the hollow tube 10', detached from the tissue 50 in case the distal end 10b has been inserted into the tissue 50 a distance being the same or greater than an inner diameter D10ci of the mouth 10c. However, it is in many cases acceptable that the surface 12 is smooth to such an extent that when performing a reference sample with a biopsy instrument 1 of the above kind, a core 51 is, during retraction of the hollow tube 10', detached from the tissue 50 in case the distal end 10b has been inserted into the tissue 50 a distance being 1,3 times or greater than an inner diameter D10ci of the mouth 10c. Moreover, it is in many cases acceptable that the surface 12 is smooth to such an extent that when performing a reference sample with a biopsy instrument 1 of the above kind, a core 51 is, during retraction of the hollow tube 10', detached from the tissue 50 in case the distal end 10b has been inserted into the tissue 50 a distance being 1,7 times or greater, or even 2 times or greater, than an inner diameter D10ci of the mouth 10c. The above applies at least for inner diameters D10ci being between 1-5mm.

It may be noted that the smallest or most superficial sample that typically may be obtained typically depends on the type of tissue and tumour being sampled. In general, more solid tissue and tumours are easier to sample and biopsies of down to 1 mm are typically possible. In mucosa it also depends on which organs the biopsy is retrieved from since the consistency also varies e.g. a comparably softer gastrointestinal vs a comparably more solid respiratory tract. Biopsies between 1-3 mm may typically be obtained in most types of tissues and tumours with high reproducibility.

As is shown in figure 4, the biopsy instrument 1 is capable of retrieving a plurality of tissue samples directly one after the other without a previous sample needs to be harvested. A first sample 51 is in a controlled manner pushed further into the hollow tube 10' towards the proximal end 10a by the core 52 of the second sample when the distal end 10b is advanced into the tissue 50. The fact that the hollow tube 10' is provided with a smooth interior surface 12 being smooth to such an extent that the core 51 adhesively by itself becomes adhered to the inside of the hollow tube 10' makes it possible to retrieve samples with a minimum of damage to the sample 51 and still allow for the cutting edge 11 and distal end 10b to be drilled into and out of the tissue 50 thereby reducing discomfort for the patient. In figure 4, a variant without an outer elongated hollow tubular member is depicted. It may be noted that a biopsy instrument including an outer elongated hollow tubular member 14, such as e.g. of the kind disclosed in figures 2a and 3a may also be used to retrieve a plurality of samples by advancing and retracting the hollow tube 10' relative to the outer elongated hollow tubular member 14 or alternatively by advancing and retracting the hollow tube 10' and the outer elongated hollow tubular member 14 together relative to the endoscope 40.

The hollow tube 10' is liquid tight and air or gas tight. However, it should be noted that the liquid and air or gas tightness is not intended to address any long-term liquid and air or gas tightness, which is typically discussed when it comes to long term storing of a liquid or a gas. The hollow tube 10' should be liquid tight and air or gas tight such that suction is provided at the interface between the inside wall of the hollow tube 10' and the core 51 of the tissue sample when the hollow tube 10' is retracted. The hollow tube 10' is liquid tight air or gas tight at least along the length of the tubular sample acquiring portion 10b' along the central geometrical axis A. The tubular sample acquiring portion 10b' has preferably an extension and is provided with said smooth surfaces 12 along a length 10b' from the distal end 10b towards the proximal end 10a, the extension 10b' having at least a length allowing for at least two, preferably at least three, reference samples of the above disclosed kind each having an insertion depth being at least equal to, or at least 1,3 times, or at least 1,7, or even 2 times the inner diameter D10ci to be acquired one after the other. In the preferred embodiment, the hollow tube 10' is air tight along the complete length from the proximal end 10a to the distal end 10b.

As is shown in figure 6, the samples 51, 52, 53, 54, 55 may be harvested in a controlled manner such that each sample 51, 52, 53, 54, 55 is still uniquely identifiable and still undamaged. This allows for the physician to keep any information provided by the stratigraphy and/or position of respective sample 51, 52, 53, 54, 55, which in turn may be used to increase the amount of data provided by the biopsy, which in turn may increase the accuracy of the diagnosis ultimately provided.

Harvesting may e.g. be performed by using a mechanical tool schematically indicated by the arrow 71 in figure 6 being inserted into and extending through the complete biopsy instrument from the proximal end 10a to the distal end 10b such that the samples 51, 52, 53, 54, 55 may securely be pushed out. As is shown in figure 7, an elongated hollow tube 10' also allows for harvesting using a burst of air at the proximal end 10a pushing the samples 51, 52, 53, 54, 55 out at the distal end 10b. This latter would require that the elongated tube 10' is sufficiently air tight such that a sufficient amount of the burst of air, or other kinds of gaseous or liquid fluids, actually pushes the samples 51, 52, 53, 54, 55 out. The burst of air may e.g. be provided by a syringe 70 being connected to the proximal end 10a of the hollow tube 10'.

The smooth inner surface 12 is preferably formed of a polymer-based material 12. The polymer-based material may e.g. be ethylene tetrafluoroethylene ETFE. It is also conceivable to use other plastic materials such as other fluoropolymers, such as polytetrafluorethylene PTFE, perfluoroalkoxy PFA, fluorinated ethylene propylene FEP.. The inner surface may also at least partially be formed of medical grade stainless steel polished to a desired smoothness.

It may be noted that the polymer-based material 12 may be provided in various different physical designs. The polymer-based material 12 may be provided in the form of an elongated tubular member. The polymer-based material 12 may be attached to an inside of an outer member. The polymer-based material 12 may be provided inside an outer member and be movable and rotatable relative to the outer member. The polymer-based material 12 may be provided as a coating inside an outer member. The various physical designs will be discussed in more detail below.

It may be noted that in the detailed description above, the design of the hollow tube 10' and the movement of the hollow tube 10' relative to the tissue 50 has been discussed as such. Other parts of the biopsy instrument 1 may be designed in several different ways to achieve the intended movement of the hollow tube 10' in suitable ways for different use scenarios. Different embodiments indicating a representative selection of some of such different ways will be disclosed in detail in the following.

In the embodiment shown in detail in figures 11 and 12, the inner elongated hollow tubular member 13 comprises a smooth interior surface 12 formed of a polymer-based material rotationally and translationally fixed relative to an inside of a support part of the inner elongated hollow tubular member 13.

As is shown in figure 12, the support part of the inner elongated hollow tubular member 13 comprises a hollow metallic wire rope 13' capable of transferring a force along the central geometrical axis A such that a movement LF, LB of the proximal end 10a along the central geometrical axis A is transferred to a movement LF, LB of the distal end 10b along the central geometrical axis A, and of transferring a torque about the central geometrical axis A such that a rotation ω and a torque T applied by a motor 31 at the proximal end 10a about the central geometrical axis A is transferred from the proximal end 10a to the distal end 10b thereby rotating the distal end 10b about the central geometrical axis A.

As is e.g. shown in figures 11 and 12, the hollow tube 10' has at a proximal end 13a thereof a connector 15 for connection to a motor 31, the connector 15 being capable of transferring said movement LF, LB along the central geometrical axis A and said rotation ω and torque T.

The hollow tube 10' further comprises an outside layer 13" arranged outside of the elongated hollow tubular member 13. The outside layer 13" may e.g. be a polymer-based shrink film.

As is indicated in figure 12, the hollow tube 10' is in accordance with a first embodiment designed and optionally also manufactured in accordance with the following.

An endtube 16 is mounted to the distal end 10b of the hollow metallic wire rope 14. The distal end 10b has been subjected to grinding. The endtube 16 is provided with a cutting edge 11. The cutting edge 11 may be sharpened. The endtube 16 may have openings 16b used in a laser welding process by which the endtube 16 is fastened to the outside of the hollow metallic wire rope 13'. The distal end of the endtube may be laser welded to the surface of the hollow metallic wire rope around the complete circumference of said rope. A base connector 17 is crimped or shrunk onto the proximal end 10a of the hollow metallic wire rope 13'. The base connector 17 is in turn designed to be connected to a connector 15, wherein the connector 15 is designed to be connected to a manoeuvring unit 30. In a sense it may be said that the base connector 17 forms part of the connector 15. The connector 15, 17 is manufactured in two main parts 15, 17 since it is advantageous to have a small and straight-forward design on the part 17 actually being attached to the hollow metallic wire rope 13'. The desired functionality concerning user friendly connectivity between the connector 15 and the manoeuvring unit 30 is then provided by the connector 15. The connection between the base connector 17 and the connector 15 is such that it is capable of transferring said force along the central geometrical axis A and of transferring said torque about the central geometrical axis A such that said rotation ω and said torque T may be transferred.

The smooth inner surface 12 is provided by an inner material is positioned inside the hollow metallic wire rope 13'. In the disclosed embodiment, the inner material is in the form of a polymer-based film, preferably a tubular polymer-based film. The inner material 13 is welded to the hollow metallic wire rope 13'. The inner material preferably has an overlength compared to the length of the hollow metallic wire rope 13' when it is positioned inside the hollow metallic wire rope 13' and is welded and fixated in its position before it is cut in flush. It may also be mentioned that it is preferred that the cutting edge 11c also is flush with the distal end 10a of the hollow tube 10'. Thereby will the smooth surface 12 extend all the way up to the distal end 10a.

An outer shrink tube is shrunk onto the outside of the hollow metallic wire rope 13'.

It may be noted that it is also conceivable that the inner material 13 shown in figure 12, is rotatable and translationally movable relative to the support part 13'. The inner material 13 could e.g. be a hollow polymer-based elongated tube 13 forming a base member 10 and having sufficient rigidity to be capable of transferring a force along the central geometrical axis A such that a movement LF, LB of the proximal end 10a along the central geometrical axis A is transferred to a movement LF, LB of the distal end 10b along the central geometrical axis A, and of transferring a torque about the central geometrical axis A such that a rotation ω and a torque T applied by a motor 31 at the proximal end 10a about the central geometrical axis A is transferred from the proximal end 10a to the distal end 10b thereby rotating the distal end 10b about the central geometrical axis A. In such a design, the hollow metallic wire rope 13' would form a stationary outer elongated hollow tubular member 14. In such a design, the distal end 10b of the inner material 13 may form the cutting edge 11.

In figure 27, there is disclosed an embodiment in which an elongated member 10 of the kind disclosed with reference to figure 12, with the inner polymer-based tube 13 being fixed inside the hollow metallic wire rope 13', is arranged inside an outer elongated hollow tubular member 14 such that it is independently rotationally and translationally movable relative to the outer elongated hollow tubular member 14.

Irrespective of the specific design of the base member 10, the outer elongated hollow tubular member 14 may be a hollow metallic wire rope. Preferably, the inner tubular member 13 is formed of a hollow metallic wire rope and the outer elongated hollow tubular member 14 is formed of a hollow metallic wire rope. Optionally, the outer elongated hollow tubular member 14 may be provided with an internal tube, such as a polymer-based tube.

Alternatively, a base member 10 of the kind disclosed with reference to figure 12, with the inner polymer-based tube 13 being fixed inside the hollow metallic wire rope 13', may be arranged inside a working channel 41 of an endoscope 40 such that it is independently rotationally and translationally movable relative to the working channel 41. In such a design, the working channel 41 of the endoscope 40 may in a sense be said to form an outer elongated hollow tubular member 14.

However, it is preferred that the outer elongated hollow tubular member 14 forms part of a biopsy instrument 1. For use with an endoscope 40, it is preferred that the biopsy instrument 1 is provided with an outer elongated hollow tubular member 14 and a base member 10 that it is independently rotationally and translationally movable relative to the outer elongated hollow tubular member 14 and that the biopsy instrument 1 is in turn inserted into the working channel 41 of the endoscope 40. With such a design, the outer elongated hollow tubular member 14 is translationally moveable and preferably also rotatable inside the working channel 41. However, this movability and rotatability is intended to be used to position the outer elongated hollow tubular member 14 relative to the endoscope 40 and relative to the tissue 50, whereas the rotation intended to make the cutting edge 11 to cut the tissue 50 is provided by rotating the base member 10 relative to the outer elongated hollow tubular member 14.

With reference to figures 15 and 16, an embodiment in which the inner elongated hollow tubular member 13 is arranged inside the outer elongated hollow tubular member 14 and is rotationally and translationally movable relative to the outer elongated hollow tubular member 14 will be described in more detail in the following. The inner elongated hollow tubular member 13 may e.g. be of the kind disclosed with reference to figure 12, with the inner material being fixed to the hollow metallic wire rope 13'. The outer elongated hollow tubular member 14 is intended to be kept stationary relative to the endoscope during the sample acquiring process. The inner elongated hollow tubular member 13 is intended to be rotated and to be advanced into the tissue 50 while the outer elongated hollow tubular member 14 remains outside the tissue 50. The distal end 14b of the outer elongated hollow tubular member 14 is provided with a stopper 19 as is shown in figure 16, The stopper 19 prevents the distal end 14b from being unintentionally advanced into the tissue 50. The stopper 19 is designed to increase the abutment surface between the distal end 14b of the outer elongated hollow tubular member 14 and the tissue 50. The stopper 19 provides this increased abutment surface by being positioned at the distal end 14b and by being designed to provide one or more bodies increasing the circumference of the distal end 14b. The stopper 19 may be an inflatable ring 19 attached to the outer elongated hollow tubular member 14. The stopper 19 may be one or more arms 19' pivotably connected to the outer elongated hollow tubular member 14. The increased abutment surface provided by stopper 19 leads to stability and works as an opposing force when the inner elongated hollow tubular member 13 is retracted whereby the sample may be removed more easily and without as much pull on the tissue surrounding the sample site. Moreover, by having an outer elongated hollow tubular member 14 which may be kept stationary relative to the endoscope during the sample acquiring process in combination with an inner elongated hollow tubular member 13 being rotationally and translationally movable relative to the outer elongated hollow tubular member 14 the outer elongated hollow tubular member 14 may be designed with a comparably close fit to the working channel 41 of the endoscope. Moreover, since the relative movement is provided between two components of an instrument being specifically designed and manufactured for interaction with each other, it is possible to provide a comparable close fit between the inner and outer elongated hollow tubular members 13, 14 and still secure that sufficient play is provided. Moreover, by being able to use a close fit, the inner and outer elongated hollow tubular members 13, 14 will in a sense support each other and prevent each other from collapsing, which in turn makes it possible to use comparably thin material thicknesses in both the outer and inner elongated hollow tubular members 13, 14. This will in turn make it possible to have an inner diameter D10ci of the distal end 13b of the inner elongated hollow tubular member 13 being comparably large for a given working channel 41 having a given interior diameter.

The inner elongated hollow tubular member 13 is capable of transferring a force along the central geometrical axis A such that a movement LF, LB of the proximal end 10a along the central geometrical axis A is transferred to a movement LF, LB of the distal end 10b along the central geometrical axis A, and of transferring a torque about the central geometrical axis A such that a rotation ω and a torque T applied by a motor 31 at the proximal end 10a about the central geometrical axis A is transferred from the proximal end 10a to the distal end 10b thereby rotating the distal end 10b about the central geometrical axis A.

The inner elongated hollow tubular member 13 has at a proximal end 13a thereof a connector 15 for connection to a motor 31, the connector 15 being capable of transferring said movement LF, LB along the central geometrical axis A and said rotation ω and torque T.

The outer elongated hollow tubular member 14 has at a proximal end 14a thereof a connector 18 for connection to a manoeuvring unit 30 such that the outer elongated hollow tubular member 14 may be moved to the intended sample site and be kept still during the sample being acquired by the advancement LF and retraction LB of the inner elongated hollow tubular member 13 while the inner elongated hollow tubular member 13 being rotated by the motor 31.

The inner elongated hollow tubular member 13 is at the distal end thereof provided with said distally facing circular cutting edge 11. The cutting edge 11 may be provided on a separate member such as an endtube 16 as discussed with reference to figure 12. However, since the inner and outer elongated hollow tubular members 13, 14 support each other and thereby may be designed with thin material thicknesses it is conceivable to use a cut distal end of the inner elongated hollow tubular member 13 as such as the cutting edge 11.

If the diameter of the mouth defined by the cutting edge is 1 mm and the rotation speed is 15000 rpm, the peripheral speed of the cutting edge will be 0.75 m/s. It is presently considered that if the peripheral speed is above about 0.40 m/s, the cutting edge will be efficient in cutting through a tissue. Such a peripheral speed enables the cutting edge to have a cutting radius of about 0.02 mm, which corresponds to a relatively blunt cutting edge. The cutting radius may be smaller. A blunt cutting edge having a cutting radius of 0.01 to 0.02 mm will be convenient from a handling perspective, since the blunt cutting edge will not easily harm a user, which by accident hits the cutting edge during handling, and still be efficient for the biopsy procedure. A cutting edge having a cutting radius of 0.001 to 0.01 mm will be more efficient in cutting tissue during the biopsy procedure. A larger diameter of the cutting edge, for example 2 mm (or 4 mm) will result in a higher peripheral speed of about 1.5 m/s (3 m/s), which is still better from the perspective of efficient biopsy procedure.

The manoeuvring unit 30 comprises in short, a housing 32, an electric motor 31 inside the housing 32, and a connector 33. The connector 33 is configured to be interconnected with the connector 15 and is connected to the motor 31 such that a torque T and rotation ω may be transferred from the motor 31 to the connector 15. The manoeuvring unit 30 also comprises one or more batteries 34a-b. The manoeuvring unit 30 may be provided with one or more buttons 35a-b. The buttons 35a-b may e.g. be used start and stop the motor 31. The manoeuvring unit 30 may be provided with one or more electric connections as exemplified by connection 36. The connection 36 may e.g. be used to provide an interface to a pedal 37, which is shown in figure 1, whereby the pedal 37 may be used to start and stop the motor 31. The user U may e.g. be given the option to vary the rotational speed by depressing/releasing the pedal 37. A connection 36 may also be used for charging the batteries 34a-b in the manoeuvring unit 30. The connection 36 and housing 32 may be configured to receive a connector 80 extending from the connection 36 as a typical connector 80 at an end of an electrical wire 81 as e.g. shown in figure 8. The connection 36 and housing 32 may be configured to receive a sub-housing 82 having a shape and size forming an extended part 32' of the housing 32. It may e.g. have the same circumferential shape and size and be attached to the end of the housing 32 as shown in figures 9 and 10. An electric wire 81 may extend from this part 32' of the housing 32. Such an extended part 32' of the housing 32 may house the batteries 34a-b. The batteries 34a-b may thereby be quickly replaceable, they may be charged separated from the housing part comprising the motor 31 and connector 33, and it is possible to use a single manoeuvring unit 30 with the motor 31 and connector 33 together with more than one extended part 32 each being provided with its own set of said one or more batteries 34a-b.

In figure 10, it is shown how the manoeuvring unit 30 is connected to the biopsy instrument 1 by the connector 15 being connected to the connector 33.

In figure 17, there is shown a telescope mechanism 90. A telescope mechanism may also be referred to as a telescope functionality. The telescope mechanism 90 may include a cover 91 at least partly but preferably completely covering the part of the biopsy instrument 1 between the access opening 41a and the manoeuvring unit 30. The telescope mechanism 90 may have an adjustable length along the axis A such that a biopsy instrument 1 of a certain length may be used in different kinds of endoscopes 40 having slightly different lengths of the working channel 41 as measured between the access opening 41a and the distal opening 41b. The telescope mechanism 90 may also provide a limit concerning a maximum extension of the distal end 10b of the elongated hollow tubular member 10 and/or a maximum extension of the distal end 14b of the outer elongated hollow tubular member 14. The telescope mechanism 90 may also be provided with a locking member 92 by which the outer elongated hollow tubular member 14 is fixable relative to the endoscope 40 once the biopsy instrument 1 has been moved to the intended sample site. The telescope mechanism 90 may also be provided with a locking member or abutment member 93 by which a maximum relative motion between the inner elongated hollow tubular member 13 and the outer elongated hollow tubular member 14 may be set, whereby a well-defined maximum sample depth may be provided for. It may be noted that in figure 17, the distal end of the endoscope 40 and the biopsy instrument 1 is for clarity reasons shown enlarged. However, in practice, the biopsy instrument 1 typically has the same diameter at the distal end portion 10b' as it has in other portions along the length of the biopsy instrument as e.g. shown in figure 19.

The telescopic mechanism 100 shown in figures 18-22 is especially configured for a biopsy instrument of the kind disclosed in figures 15-16, i.e. a biopsy instrument having a non-rotating outer elongated hollow tubular member 14 and an inner elongated hollow tubular member 13 being rotatably arranged inside the outer elongated hollow tubular member 14. A proximal end of the telescopic mechanism 100 is connected to the motor 30 and a distal end of the telescopic mechanism 100 is connected to the endoscope 40. Different parts of the telescopic mechanism 100 is connected to different parts of the biopsy instrument 1 as will be disclosed in more detail in the following.

The telescopic mechanism 100 comprises a base sleeve 110. The base sleeve 110 is at a distal end thereof provided with a connector 111 by which the base sleeve 110 is configured to be connected to an insertion opening 41a of an endoscope 40. The motor 30 is configured to be connected to a proximal end of the base sleeve 110. The base sleeve 110 has a fixed length.

The telescopic mechanism 100 further comprises an inner sleeve 120 which is slidably arranged inside the base sleeve 110. The inner sleeve 120 is connected to the outer elongated hollow tubular member 14 such that a sliding motion of the inner sleeve 120 in a distal direction relative to the base sleeve 110 results in that the outer elongated hollow tubular member 14 is moved in a distal direction relative to the endoscope. The telescope mechanism 100 further comprises a first ring member 115 which is movably arranged around the base sleeve 110. The first ring member 115 is slidable back and forth along the base sleeve 110. It may be said to control the length of the outer elongated hollow tubular member 14 at the distal end of the endoscope 40. The first ring member 115 is provided with a connector 116, which in the disclosed embodiment is a screw and wedge, by which the first ring member 115 may be connected to the inner sleeve 120. In the disclosed embodiment, the screw is positioned in a threaded hole in the first ring member 115 and pushes a wedge into contact with the inner sleeve 120 when the screw is screwed into the threaded hole of the first ring member 115, which may be said to adjust the length of the outer elongated hollow tubular member 14 out of the endoscope distally. The connector 116 extends through a through-going long hole 112 formed in the wall of the base sleeve 110. By moving the first ring member 115 relative to the inner sleeve 120 to a desired location and connecting the first ring member 115 to the inner sleeve 120 at the desired location by activating the connector 116, in combination with the fact that the connector 116 extends through a long hole 112, it is possible to define to what extent the outer elongated hollow tubular member 14 may be moved out of the distal opening 41a of the endoscope 40. When the connector 116, which is connected to the inner sleeve 120 and which extends through the long hole 112, hits the distal end of the long hole 112, the connector 116 and thus also the first ring member 115 and the inner sleeve 120 is prevented from moving any further in the distal direction relative to the base sleeve 110.

The telescopic mechanism 100 further comprises a central sleeve 130 which is slidably arranged inside the inner sleeve 120. The central sleeve 130 is connected to the inner elongated hollow tubular member 13 such that a sliding motion of the central sleeve 130 in a distal direction relative to the inner sleeve 120 results in that the inner elongated hollow tubular member 13 is moved in a distal direction relative to the outer elongated hollow tubular member 14. The inner elongated hollow tubular member 13 is rotatable inside the central sleeve 130. In the preferred embodiment, the inner elongated hollow tubular member 13 extends in a bore 131 through the central sleeve 130, the bore 131 having a diameter such there is a play between the inside of the bore 131 and the inner elongated hollow tubular member 13.

The telescope mechanism 100 further comprises a second ring member 125 which is movably arranged around the base sleeve 110. The second ring member 125 is slidable back and forth along the base sleeve 110. The second ring member 125 is provided with a connector 126, which in the disclosed embodiment is a screw and wedge, by which the second ring member 125 may be connected to the central sleeve 130. In the disclosed embodiment, the screw is positioned in a threaded hole in the second ring member 125 and pushes a wedge into contact with the central sleeve 130 when the screw is screwed into the threaded hole of the first ring member 115. The connector 126 extends through a through-going long hole 113 formed in the wall of the base sleeve 110 and through a through-going long hole 121 in the inner sleeve 120. By moving the second ring member 125 relative to the central sleeve 130 to a desired location and connecting the second ring member 125 to the central sleeve 130 at the desired location by activating the connector 126, in combination with the fact that the connector 126 extends through the long hole 121 in the inner sleeve 120, it is possible to define to what extent the inner elongated hollow tubular member 13 may be moved out of the outer elongated hollow tubular member 14.. When the connector 126, which is connected to the central sleeve 130 and which extends through the long hole 121, hits the distal end of the long hole 121, the connector 126 and thus also the second ring member 125 and the central sleeve 120 is prevented from moving any further in the distal direction relative to the inner sleeve 120.

The telescopic mechanism 100 further comprises a connector 135 configured to interconnect the central sleeve 130 and the inner sleeve 120 at a desired relative position as seen along the direction along which the central sleeve 130 is slidable relative to the inner sleeve 120. In the disclosed embodiment the connector 135 is connected to the central sleeve 130 at a fixed position along said sliding direction. The connector 135 extends through a through-going long hole 122 formed in the wall of the inner sleeve 120 such that the connector 135 is accessible to a user and such that the central sleeve 130 may be slid relative to the inner sleeve 120 without the connector 135 preventing such sliding motion. The connector 136 is configured to be activated and interconnect the inner sleeve 120 to the central sleeve 130. In the disclosed embodiment, the connector 136 is screwed further into a threaded hole in the central sleeve 130 such that the head of the screw interacts with the walls of the inner sleeve 120 on the sides of the long hole 122.

It may be noted that it is conceivable that the telescopic mechanism 100 may comprise the complete set of functionalities disclosed above and as shown in e.g. figures 18-21. However, it is also conceivable that for certain applications it is desired that only one or two of the above described functionalities are present.

It is e.g. conceivable that for some applications it is preferred that it is possible to adjust the maximum length by which the outer elongated hollow tubular member 14 extends out of the distal opening 41b of the endoscope 40 in combination with a possibility to adjust the maximum length by which the inner elongated hollow tubular member 13 may be moved out of the outer elongated hollow tubular member 14. Such a set-up would typically be useful when there is a desire to perform a biopsy as indicated in figures 3 and 4.

In an alternative embodiment there is only one setting available, namely the possibility to interconnect the inner sleeve 120 and the central sleeve 130. Such a set-up would typically be useful when there is a desire to perform a biopsy as indicated in figures 13a-b. The user would set a fixed distance by which the inner elongated hollow tubular member 13 extends out of the outer elongated hollow tubular member 14 and thereafter the inner and outer elongated hollow tubular members 13, 14 would be moved together relative to the distal opening 41b of the endoscope 40, taking a superficial sample from the surface of the organ wall as e.g. shown in figures 13a-b and 14a-c.

It may in this context also be noted that the telescopic mechanism 90, 100 may be a separate part, i.e. being separate from and connectable to the endoscope 40, the biopsy instrument 1, and the motor 31. Alternatively, it may e.g. form part of the biopsy instrument 1 and as such have an interface for connection to a motor 31 and optionally also have an interface for connection to an endoscope 40. In figure 18, there is schematically disclosed how a manoeuvring unit 30 comprising a motor 31 is connected to the telescopic mechanism. Alternatively, the telescopic mechanism 90, 110 may be connectable to a manoeuvring unit 30 comprising a motor 31 via a drive wire 39 such as disclosed in figure 27.

In figure 22 there is schematically shown a design where the telescopic mechanism 100 is separate from the biopsy instrument 1. The telescopic mechanism 100 may be an integral part of the manoeuvring unit 30 but may alternatively be a separate part being connectable to the manoeuvring unit 30. The biopsy instrument 1 comprises an interface for connection to the telescopic mechanism. The interface comprises a first connection member 13e which is connected to the inner elongated hollow tubular member 13 and which is configured to be connected to the central sleeve 130 of the telescopic mechanism 100. The interface comprises a second connection member 14e which is connected to the outer elongated hollow tubular member 14 and which is configured to the connected to the inner sleeve 120.

The biopsy instrument 1 may in actual biopsy sampling be used in accordance with a number of different methods. It may e.g. be used in accordance with one method where the biopsy instrument is used as shown in figures 3-5, i.e. where the distal end 10b is advanced a distance into the tissue 50 and thereafter is retracted. However, the biopsy instrument 1 may in accordance with another method be used to move along the surface of the tissue 50 from which the biopsy is to be obtained as e.g. shown in figures 13a-c and 14a-c. In the user method shown in figures 3a-b and 4, the distal end 10b is fully inserted into the tissue 50 in the sense that the distal end 10b is inserted with the complete circumference C inserted into the tissue 50 whereby an adhesive force larger than breaking force needed to detach the core from the tissue is formed. In the method shown in figures 13a-c and 14a-c, the distal end 10b is only partly inserted into the tissue 50 in the sense that the distal end 10b is inserted with only a portion of the complete circumference C being inserted into the tissue 50, as is shown in figure 14a. In figure 14a about half of the circumference C - the bottom half in figure 14a - is inserted into the tissue 50. As is shown in figures 13a-b, the biopsy instrument 1 is in this method moved along the surface of the tissue 50 and cuts basically a continuous or at least semi-continuous groove 57 in the surface of the tissue 50.

The distal end of the base member is rotated with high speed (13 000 rpm or more). This means that the distal end of the base member, which extends out of the elongated tubular member will be stabilized so that deviations from a straight path will be counteracted. This is an advantage if the tissue is softer/harder at different locations, as often is the case with cancer tumours. The sample will be taken according to a substantially straight path independently of any deviations in softness/hardness of the tissue. This relates amongst others to the embodiments according to figures 3-5 and the embodiments according to figures 13a-14c.

In the user method shown in figures 3a-b and 4, a telescope mechanism, such as the telescope mechanism 100, may be set such that the inner elongated hollow tubular member 13 is rotatable relative to the outer elongated hollow tubular member 14 and such that the inner elongated hollow tubular member 13 is translationally movable relative to the outer elongated hollow tubular member 14 between a proximal most position in which the inner elongated hollow tubular member 13 is completely hidden inside the outer elongated hollow tubular member 14 and a distal most position in which the inner elongated hollow tubular member 13 extends a predetermined maximum distance out of the outer elongated hollow tubular member 14. The telescopic mechanism, such as the telescope mechanism 100, may be set such that the outer elongated hollow tubular member 14 is initially movable relative to the working channel 41 of the endoscope 40 and that once the intended position of the distal end 14b the outer elongated hollow tubular member 14 has been reached, the position of the outer elongated hollow tubular member 14 may be fixed relative to the endoscope 40.

In the user method shown in figures 13a-c and 14a-c, a telescope mechanism, such as the telescope mechanism 100, may be set such that the inner elongated hollow tubular member 13 is rotatable relative to the outer elongated hollow tubular member 14 and such that the inner elongated hollow tubular member 13 is initially translationally movable relative to the outer elongated hollow tubular member 14 between a proximal most position in which the inner elongated hollow tubular member 13 is completely hidden inside the outer elongated hollow tubular member 14 and a distal most position in which the inner elongated hollow tubular member 13 extends a predetermined maximum distance out of the outer elongated hollow tubular member 14, whereby the inner elongated hollow tubular member 13 is hidden inside the outer elongated hollow tubular member 14 as the biopsy instrument is inserted into the working channel 41 and is positioned relative to the tissue 50 whereafter the inner elongated hollow tubular member 13 is moved to the distal most position and fixed in the distal most position, such that the outer elongated hollow tubular member 14 may be moved along the tissue 50 with the distal end 13b of the inner elongated hollow tubular member 13 extending a predetermined distance, preferably fixed at this predetermined distance, out of the outer elongated hollow tubular member 14 and with the inner elongated hollow tubular member 13 rotating relative to the outer elongated hollow tubular member 14. In figure 13 it is indicated how the user may move the telescope mechanism 101 relative to the endoscope 40 such that the inner elongated hollow tubular member 13 and the outer elongated hollow tubular member 14 move together along the surface of the tissue.

In those cases, the base member 10 is flexible and the elongated hollow tubular member 14 is flexible, the base member 10 preferably rotates at a rotational speed of at least 13 000 rpm. Preferably the rotational speed is between 13 000 rpm and 25 000 rpm, and more preferably between 13 000 rpm and 20 000 rpm.

In figures 23 and 24 there is disclosed a variant of the biopsy instrument 1, where the outer elongated hollow tubular member 14 is a rigid hollow needle 214. The inner elongated hollow tubular member 13 is also a rigid hollow needle 213. As shown in figure 23, the inner rigid hollow needle 213 is configured to be positioned inside the outer rigid hollow needle 214. As shown in figure 24, the inner rigid hollow needle 213 has a length sufficient for it to be able to extend out of the distal opening of the outer rigid hollow needle 214. When handling the inner rigid hollow needle 213 and the outer rigid hollow needle 214, the inner rigid hollow needle 213 is preferably retracted such that it does not extend out of the distal opening of the outer rigid hollow needle 214 as shown in figure 25. In figure 25, the inner rigid hollow needle 213 and the outer rigid hollow needle 214 are about to be positioned into a manoeuvring unit 200 used to manoeuvre the inner rigid hollow needle 213 and the outer rigid hollow needle 214 in order to acquire a biopsy. The outer rigid hollow needle 214 may have an oblique end facilitating insertion of the outer rigid hollow needle 214 into the tissue to be sampled.

Moreover, it is also conceivable to provide an inner stylet inside the inner rigid hollow needle 213. The inner stylet may e.g. be provided with an oblique solid tip corresponding to the tip of the outer rigid hollow needle 214. The inner stylet may be used to cover the mouth of the inner rigid hollow needle 213 when biopsy instrument 1 is inserted into tissue to be sampled and to be removed partially or completely prior to rotation and/or insertion of said the inner rigid hollow needle 213 into the tissue...

Such a design with an inner stylet may be used in accordance with the following; the biopsy instrument 1 is moved, such as inserting the biopsy instrument 1 into the tissue through the skin or via a body cavity, to the sample site, with the inner stylet being positioned such that it during this movement of the biopsy instrument 1 closes the mouth of the inner rigid hollow needle. Thereafter, the inner stylet is moved in a proximal direction such that the mouth of the inner rigid hollow needle 213 is opened. The inner stylet is moved in the proximal direction at least a distance being sufficient to open up a distal portion of the inner rigid hollow needle 213 where the distal portion has a sufficient length to allow a sufficient amount of tissue to be retrieved into the inner rigid hollow needle 213. Thereafter, the inner rigid hollow needle 213 is advanced (and simultaneously being rotated) in the distal direction relative to the outer rigid hollow needle 214 and the sample is acquired. The inner rigid hollow needle 213 preferably rotates at a rotational speed of at least 3 000 rpm. Thereafter, the inner rigid hollow needle 213 is retracted back into the outer rigid hollow needle 214 and the biopsy instrument 1 is retracted from the sample site, preferably while still being rotated. It may be noted that it is preferred that the inner stylet is moved in the proximal direction before the inner rigid hollow needle 213 is advanced but that it is sufficient that the inner stylet is moved in the proximal direction at the latest simultaneously as the inner rigid hollow needle 213 is being retracted back into the outer rigid hollow needle 214 such that the inner stylet does not push the sample inside the inner rigid hollow needle 213 out of the inner rigid hollow needle 213. After the biopsy instrument 1 has been removed from the sample site, the inner stylet may be used for harvesting the sample from the inner rigid hollow needle 213 by moving the inner stylet in the distal direction such that the inner stylet pushes the sample out of the inner rigid hollow needle 213. The inner stylet may be rigid. The inner stylet may be flexible and by guided by the inner rigid hollow needle. This embodiment may also be used for taking several consecutive samples as shown in figures 4-6, whereby the stylet is completely removed or retracted at least so far that the samples will have place to accumulate inside the rigid hollow needle 213.

It may be noted that the use of an inner stylet may also be applicable for a flexible biopsy instrument 1 configured for use with an endoscope 40. In such a case the inner stylet is also flexible and is guided by the inner elongated hollow tubular member 13.

As shown in figure 23, the inner rigid hollow needle 213 comprises an interface section 213e and the outer rigid hollow needle 214 also comprises an interface section 214e.

In figures 26a-b, there is schematically shown example of a manoeuvring unit 200 suitable for making use of a biopsy instrument 1 of the basic type disclosed in figures 23 and 24.

Figure 26a discloses the needles positioned in the manoeuvring unit 200 and in a state ready to acquire a biopsy sample.

Figure 26b discloses schematically operation of a handle 210 of the manoeuvring unit 200 to acquire a biopsy sample.

In more detail, the manoeuvring unit 200 comprises a base member 201 supporting the different components of the manoeuvring unit 200. The manoeuvring unit 200 comprises a support 202 configured to interact with the interface section 214e of the outer rigid hollow needle 214 and keep the outer rigid hollow needle 214 in position. Preferably, the outer rigid hollow needle 214 is kept fixed relative to the manoeuvring unit 200, i.e. the outer rigid hollow needle 214 is not moveable in the longitudinal direction and it is not rotatable relative to the manoeuvring unit 200.

The manoeuvring unit 200 further comprises a sliding member or sled 202 configured to interact with the interface section 213e of the inner rigid hollow needle 213. The sled 203 also includes a motor 30 configured to rotate the inner rigid hollow needle 213 relative to the manoeuvring unit 200 and relative to the outer rigid hollow needle 14. The sled 203 is configured to be moved back and forth relative to the support 201 such that a distal end of the inner rigid hollow needle 213 may extend out the distal end of the outer rigid hollow needle 214 similarly as shown in figure 24 and such that it may be retracted again such that the distal end of the inner rigid hollow needle 213 is retracted back into the outer rigid hollow needle 214 such that the distal end of the inner rigid hollow needle 213 no longer extends out of the distal end of the outer rigid hollow needle 214. These manoeuvres of insertion and/or retraction may be manually or may be automated and electrically controlled by one or more buttons on the manoeuvring unit 200.

The sled 202 may be manoeuvred in the movement back and forth e.g. by a linkage 204 connected to a handle 205. By manoeuvring the handle 205 relative to the support 201, the sled 202 will be affected via the linkage 204. In a preferred embodiment, the manoeuvring unit 200 may comprise a second handle being fixed relative to the support 201, and the handle 205 shown in the figures 26a-b may be moved towards such a fixed handle. For reasons of clarity, such fixed handle has been omitted.

The biopsy instrument 1 of figures 23-24 is designed to be positioned inside the manoeuvring unit 200 such that the interface 214e of the outer rigid hollow needle 214 interacts with the support 202 and the interface 213e of the inner rigid needle 213 interacts with the sled 203 and the motor 30 on the sled 203. The manoeuvring unit 200 is configured to thereafter be closed by closing or placing a lid 206 over the interface sections 213e and 214e of the inner and outer rigid hollow needles 213, 214 and the associated components 202, 203 of the manoeuvring unit 200. The lid 206 may be hinged relative to the base member 201. It may be connected to the base member 201 in other suitable manners, such as being slidably connected to the base member 201, being completely removable using a snap-fit connection or the like, etc.

The manoeuvring unit 200 is provided with a motor control, which e.g. may be a switch or button operated by the user or which may be an automatic controller connected to the manoeuvring of the sled 202 such that when the user begins to move the sled 202 the motor controller starts the motor 30 such that the inner rigid hollow needle 213 begins to rotate such that it rotates through-out the sample acquiring process.

After the sample has been acquired, the inner rigid hollow needle 213 is retracted into the outer rigid hollow needle 214 and the manoeuvring unit 200 is moved such that the inner and outer rigid hollow needles 213, 214 are moved out of the tissue being sampled.

The interface section 213e of the inner rigid hollow needle 213 may be provided with a plug or the like being capable of closing the proximal end of the inner rigid hollow needle 213. By the provision of such a plug, air trapped inside the inner rigid hollow needle 213 between the plug at the proximal end and the tissue at the distal end will form an air-cushion preventing excessive amounts of tissue being accumulated inside the inner rigid hollow needle 213. Alternatively, such a plug may be replaced by a mechanical blocking member positioned inside the inner rigid hollow needle 213. Such a mechanical blocking member is preferably inserted from the proximal end of the inner rigid hollow needle 213. The mechanical blocking member may, but need not, provide an air-tight or partially air-tight connection with the inside to the inner rigid hollow needle 213. It may be noted that this provision of an air-plug or mechanical blocking member is not limited to the design of the biopsy instrument shown in figures 23-26a-b. The concept of having an air-plug or mechanical blocking member is applicable to all the biopsy instruments disclosed.

The blocking member may during insertion be positioned such that it blocks or closes the mouth of the inner rigid hollow needle 213 or inner elongated hollow tubular member 213.

In figure 27, there is disclosed a variant of the biopsy instrument 1 configured to be used in combination with an endoscope 40 as e.g. disclosed in figure 1a. Unless explicitly contradicted by the disclosure below, the biopsy instrument 1 and the kit of parts is of the kind discussed above, especially with reference to figures 1-22.

The biopsy instrument 1 comprises a manoeuvring unit 30 comprising a motor 31. In the embodiment disclosed in figure 27, the manoeuvring unit 30 is a separate box configured to be positioned on a shelf or the like. The manoeuvring unit 30 may hold a power source and/or may be connected to a power source. The manoeuvring unit 30 may e.g. include batteries and/or may be connected to mains 38.

The biopsy instrument 1 comprises a telescopic mechanism. The telescopic mechanism is in this embodiment connected to the inner elongated hollow tubular member 13 and the outer elongated hollow tubular member 14 such that they from the user's perspective are an integral part, which is used, and typically also disposed of, as a single part. Alternatively, the telescopic mechanism may be a separate part connectable to the inner elongated hollow tubular member 13 and the outer elongated hollow tubular member 14.

The telescopic mechanism may e.g. be a telescopic mechanism 100 of the kind disclosed in detail with reference to figures 18-22. In figure 27, the telescope mechanism is a telescope mechanism 101 of the kind disclosed in more details in figures 28-30. The telescopic mechanism 101 is at a distal end thereof provided with a connector 111 by which the telescopic mechanism 101 is configured to be fixedly connected to an insertion opening 41a of an endoscope.

The telescopic mechanism 101 is at a proximal end thereof provided with a connector 15 configured to connect the base member 10 to the motor 31. In the embodiment shown in figure 27, the motor 31 is connected to the connector 15 via a drive wire 39. The drive wire 39 is preferably flexible. The drive wire 39 comprises an inner drive wire 39i which transmits rotation and torque from the motor 31 to the base member 10 and an outer casing 39c which is stationary relative to the manoeuvring unit 30 and stationary relative to a handle 102 of the telescopic mechanism 101.

With reference to figures 28-30, the telescope mechanism 101 comprises a handle 102. The handle 102 is connected to the base member 10 such that the base member 10 is rotatable relative to the handle 102. The handle 102 is connected to the base member 10 such that when the handle 102 is translated along the geometrical axis A, the base member 10 will also be translated along the geometrical axis A. Preferably, the base member 10 is translationally coupled to the handle 102 such that the translational movement of the handle 102 relative to the connector 111 along the geometrical axis A provides a corresponding, and more preferably the same, translational movement of the base member 10 relative to the connector 111.

The handle 102 also comprises a connection to the drive wire 39 such that the inner drive wire 39i may transmit rotation and torque from the motor 31 to the base member 10 and such that the outer casing 39c is stationary relative to the handle 102.

The telescope mechanism 101 further comprises an intermediate part 103. The intermediate part 103 may also be referred to as a base member adjuster. The handle 102 is translationally movable relative to the intermediate part 103. As is shown in figure 29, the handle 102 is hollow and is capable of receiving the intermediate part 103. The intermediate part 103 is slidably received in the handle 102. In figures 27-29, the intermediate part 103 and the handle 102 are shown in an extended position; extended in the sense that intermediate part 103 extends a maximum distance out of the handle 102.

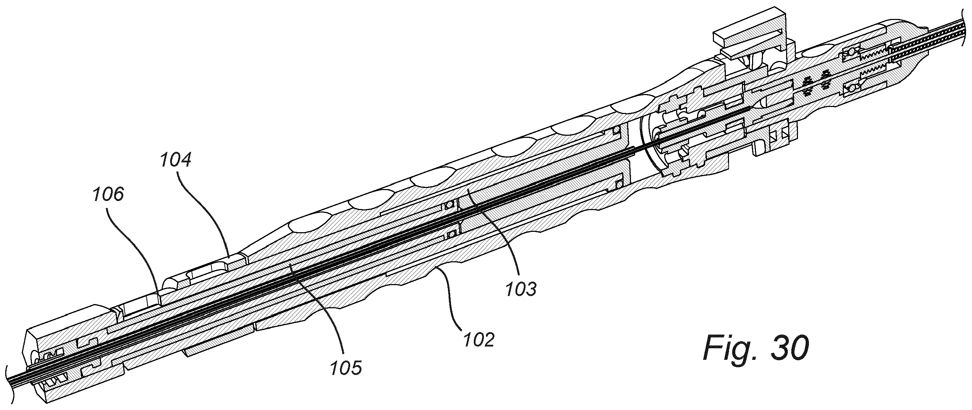

In figure 30, the intermediate part 103 is received in the handle 102. Since, the intermediate part 103 being received in the handle 102 results in that the handle 102 has moved closer to the connector 111, the base member 10, which is connected to the handle 102, has been moved in the distal or forward direction relative to the connector 111. Thus, the movement of the handle 102 towards the connector 111 relative to the intermediate part 103 causes the base part 10 to be moved such that it is advanced relative to the endoscope 40, and optionally also relative to the outer sheath 14.

The telescope mechanism 101 further comprises an adjustment member 104. The adjustment member 104 is slidably received on the intermediate part 103, such that the adjustment member 104 may be slid along the central geometrical axis A relative to the intermediate member 103. The adjustment member 104 is provided with a locking member 104a configured to lock the adjustment member 104 at different positions along the central geometrical axis A relative to the intermediate member 103. The handle 102 is configured to receive the intermediate part 103 until the handle 102 abuts the adjustment member 104. Thereby, there is provided a mechanism allowing the operator to move the base member 10 while still controlling the maximum distance the base member 10 may be advanced.

In figure 28, the adjustment member 104 is in its forward most position, i.e. in a position in which the handle 102 may be moved a maximum distance relative to the intermediate part 103 until the handle 102 abuts the adjustment member 104.

The telescope mechanism 101 further comprises an end part 105. The end part 105 may also be referred to as an outer elongated hollow tubular member adjuster.

The end part 105 is translationally movable relative to the intermediate part 103. As is shown in figure 29, the intermediate part 103 is hollow and is capable of receiving the end part 105. The end part 105 slidably received in the intermediate part 103. In figures 27-29, the intermediate part 103 and the end part 105 are shown in an extended position; extended in the sense that end part 105 extends a maximum distance out of the intermediate part 103.

In figure 30, the end part 105 is received in the intermediate part 103, which results in that the intermediate part 103 has moved closer to the connector 111.

The intermediate part 103 is connected to the outer elongated hollow tubular member 14 such that when the intermediate part 103 is translated along the geometrical axis A, the outer elongated hollow tubular member 14 will be also be translated along the geometrical axis A. Preferably, the outer elongated hollow tubular member 14 is translationally coupled to the intermediate part 103 such that the translational movement of the intermediate part 103 relative to the connector 111 along the geometrical axis A provides a corresponding, and more preferably the same, translational movement of the outer elongated hollow tubular member 14 relative to the connector 111. The end part 105 is provided with a channel 107 extending through the end part 105 along the central geometrical axis A. The channel 107 allows the outer elongated hollow member 14 to slidably extend through the end part 105.

Thus, when the end part 105 is received in the intermediate part 103, the outer elongated hollow tubular member 14 has moved in the distal or forward direction relative to the connector 111. Thus, the movement of the intermediate part 103 towards the connector 111 relative to the end part 105 causes the outer elongated hollow tubular member 14 to be moved such that it is advanced relative to the endoscope 40.

The telescope mechanism 101 further comprises an adjustment member 106.

The adjustment member 106 may be slidably received on the end part 105, such that the adjustment member 106 may be slid along the central geometrical axis A relative to the end part 105. The adjustment member 106 is provided with a locking member 106a configured to lock the adjustment member 106 at different positions along the central geometrical axis A relative to the intermediate member 103.

The intermediate part 103 is in one variant configured to receive the end part 105 with the adjustment member 106 being fixedly connected to the intermediate part 103, as is best shown in figure 30. Thereby, there is provided a mechanism for locking the intermediate part 103 and the end part 105 at different relative positions and thereby also locking the outer elongated hollow tubular member 14 relative to the connector 111, and thereby also relative to the endoscope 40.

The intermediate part 103 is in one variant configured to receive the end part 105 until the intermediate part 103 abuts the adjustment member 106. Thereby, there is provided a mechanism for allowing the operator to move the outer elongated hollow tubular member 14 while still controlling the maximum distance the outer elongated hollow tubular member 14 may be advanced. In this variant, the adjustment member 106 is separated from the intermediate part 103.

In the embodiments of telescope mechanism disclosed in figures 17-22 and 27-30, the drive, such as the drive wire, is aligned with the base member 10.

However, in figures 31-32, there is disclosed a variant in which the drive, such as a drive wire 39, is connected offset to the base member 10. This allows the base member 10 to extend all the way through the telescope mechanism such that it is accessible at the proximal end of the telescope mechanism. This may e.g. be useful for application of an under-pressure. In such a case, the base member 10 comprises preferably an inner hollow elongated tubular member 13, preferably being liquid or gas-tight such that an under-pressure may be applied via a connector 108 at the proximal end of the telescope mechanism.

The handle 102 is connected to the base member 10 such that the base member 10 is rotatable relative to the handle 102. The handle 102 is connected to the base member 10 such that when the handle 102 is translated along the geometrical axis A, the base member 10 will be also be translated along the geometrical axis A. Preferably, the base member 10 is translationally coupled to the handle 102 such that the translational movement of the handle 102 relative to the connector 111 along the geometrical axis A provides a corresponding, and more preferably the same, translational movement of the base member 10 relative to the connector 111.

The handle 102 also comprises a connection to the drive wire 39 such that the inner drive wire 39i may transmit rotation and torque from the motor 31 to the base member 10 and such that the outer casing 39c is stationary relative to the handle 102. In this variant, the handle 102 also comprises a gear mechanism 109 connected between the connection to the drive wire 39 and the base member 10, such that the drive wire 39 is connected offset relative to the central geometrical axis A.

It may also be noted that the different variants of the biopsy instruments 1 may also be used for additional purposes. The inner hollow elongated tubular member 13, irrespective of if it is rigid or flexible, may be used as an introduction channel for the introduction of a guide wire. The outer hollow elongated tubular member 14, irrespective of if it is rigid or flexible, may be used as an introduction channel for the introduction of a guide wire. The guide wire may e.g. be used to insert a stent, a balloon, camera, injection tube or the like. The guide wire may also be used to insert a marker, such as a marker being visible on an X-ray image. The biopsy instrument 1 would in such a case typically be used in accordance with the following: first the instrument is inserted into the tissue and optionally a sample is also acquired; thereafter one of the elongated hollow tubular members 13, 14 is optionally removed completely (if a sample has been acquired, the inner hollow elongated tubular member 13 is removed such that the sample may be harvested); thereafter the guidewire is inserted via a part of the biopsy instrument 1 still being inserted to the intended position; thereafter all parts of the biopsy instrument is retracted while the guidewire remains extending to the intended position; thereafter the stent, balloon, marker is inserted or activated; and finally the guidewire is also retracted.

## Claims

1. Kit of parts comprising
a biopsy instrument (1), and
a manoeuvring unit (30) comprising a motor (31),
wherein the biopsy instrument (1) comprises
an outer elongated hollow tubular member (14) which extends from a proximal end (14a) to a distal end (14b) along a central geometrical axis (A), and
a base member (10) which extends from a proximal end (10a) to a distal end (10b) along the central geometrical axis (A), wherein a distal end portion (10b') of the base member (10) is shaped as an elongated hollow tube (10'), the elongated hollow tube (10') at the distal end (10b) of the base member (10) being intended to be at least partly inserted into a tissue (50) from which a biopsy is to be obtained,
wherein the base member (10) is arranged inside the outer elongated hollow tubular member (14) and is independently rotationally and translationally movable relative to the outer elongated hollow tubular member (14),
wherein the base member (10) is capable of transferring a force along the central geometrical axis (A) such that a movement (LF, LB) of the proximal end (10a) of the base member (10) along the central geometrical axis (A) is transferred to a movement (LF, LB) of the distal end (10b) of the base member (10) along the central geometrical axis (A), and of transferring a torque about the central geometrical axis (A) such that a rotation (ω) and a torque (T) applied by a motor (31) at the proximal end (10a) of the base member (10) about the central geometrical axis (A) is transferred from the proximal end (10a) of the base member (10) to the distal end (10b) of the base member (10) thereby rotating the distal end (10b) of the base member about the central geometrical axis (A),
wherein the elongated hollow tube (10') is capable of being advanced out of a distal end (14b) of the outer elongated hollow tubular member (14) and to be retracted back into the outer elongated hollow tubular member (14) by a movement of (LF, LB) of the proximal end (10a) of the base member (10) along the central geometrical axis (A), while being rotated inside and relative to the outer elongated hollow tubular member (14) about the central geometrical axis (A) by the motor (31) applying a rotation (ω) and a torque (T) at the proximal end (10a) of the base member (10),
wherein the elongated hollow tube (10') is provided with a distally facing circular cutting edge (11) defining a mouth (10c) of the distal end (10b) of the elongated hollow tube (10'), and
wherein the elongated hollow tube (10') has, at a distal portion (10b') of the elongated hollow tube (10'), a hollow elongated tubular sample acquiring portion (10b') having a smooth interior surface (12),
wherein the proximal end (10a) of the base member (10) is configured to be connected to the motor (31) such that rotation (ω) and torque (T) may be applied by the motor (31) to the proximal end (10a) of the base member (10) and be transferred by the base member (10) to the elongated hollow tube (10) at the distal end (10b) of the base member (10), and wherein the base member (10) is flexible and the outer elongated hollow tubular member (14) is flexible,
**characterized in that** the motor (31) is configured to provide a rotation (ω) of the elongated hollow tube (10'), at a rotational speed of at least 13 000 rpm, while the elongated hollow tube (10') is advanced out of the distal end (14b) of the outer elongated hollow tubular member (14) and is retracted back into the outer elongated hollow tubular member (14), inside and relative to the outer elongated hollow tubular member (14) about the central geometrical axis (A) by applying rotation (ω) and torque (T) to the proximal end (10a) of the base member (10).

2. Kit of parts according to claim 1, wherein the rotational speed is between 13 000 rpm and 25 000 rpm, preferably between 13 000 rpm and 20 000 rpm.

3. Kit of parts according to claim 1 or 2, wherein the interior or outer surface of the sample acquiring portion (10b') is liquid tight, wherein preferably the whole length of the interior or outer surfaces of the base member 10) is liquid tight.

4. Kit of parts according to claim 3, wherein the distally facing circular cutting edge (11) is shaped and the smooth interior surface (12), preferably a liquid tight smooth interior surface, connects to the cutting edge (11) such that the smooth interior surface (12) extends, as seen along the central geometrical axis (A), to a most distal part of the cutting edge (11).

5. Kit of parts according to any one of claims 1-4, wherein the smooth interior surface (12) is smooth to such an extent that when a reference biopsy is to be acquired, the cutting edge (11) and the distal end (10b) of the elongated hollow tube (10') is configured to be advanced along the central geometrical axis (A) into a tissue (50) while being rotated (ω, T) by being motor driven at its proximal end (10a) at a rotational speed of at least 13 000 rpm and thereby cut a core (51) of the tissue (50) which, due to the advancement (LF) of the elongated hollow tube (10'), enters relative to the elongated hollow tube (10') through the mouth (10c) into the sample acquiring portion (10b') of the elongated hollow tube (10') with a circumferential outer surface of the core (51) at least partly abutting the smooth interior surface (12) of the sample acquiring portion (10b'), where-after the elongated hollow tube (10') is retracted from the tissue (50) while being rotated (ω, T) at a rotational speed of at least 13 000 rpm by being motor driven at its proximal end (10a) whereby the core (51) of the tissue (50) is detached from the tissue (50) by a pulling force due to the retraction (LB) of the elongated hollow tube (10') and due to an adhesive force formed at an interface between the smooth interior surface (12) and the circumferential outer surface of the core (51) which force keeps the core (51) inside the sample acquiring portion (10b') having the smooth interior surface (12).

6. Kit of parts according to any one of claims 1-5, wherein the smooth inner surface has a surface roughness with an Ra value of less than 1,5µm, preferably less than 1 µm, when formed of steel, such as a medical grade stainless steel, and less than 6 µm, such as between 1 µm and 6 µm, when formed of a polymer-based material.

7. Kit of parts according to any one of claims 1-6, wherein the smooth inner surface (12) is formed of a polymer-based material.

8. Kit of parts according to any one of claims 1-7, wherein the base member (10) comprises an inner elongated hollow tubular member (13) extending from the proximal end (10a) to the distal end (10b) of the base member (10).

9. Kit of parts according to claims 7 and 8, wherein the polymer-based material forming the smooth inner surface (12) is provided as a film, preferably a tubular film, which is inserted into the inner elongated hollow tubular member (13) and which is attached to an inside surface of the inner elongated hollow tubular member (13).

10. Kit of parts according to claim 8 or 9, wherein the inner elongated hollow tubular member (13) comprises a hollow metallic wire rope capable of transferring a force along the central geometrical axis (A) such that a movement (LF, LB) of the proximal end (10a) along the central geometrical axis (A) is transferred to a movement (LF, LB) of the distal end (10b) along the central geometrical axis (A), and of transferring a torque about the central geometrical axis (A) such that a rotation (ω) and a torque (T) applied by the motor (31) at the proximal end (10a) about the central geometrical axis (A) is transferred from the proximal end (10a) to the distal end (10b) thereby rotating the distal end (10b) about the central geometrical axis (A).

11. Kit of parts according to any one of claims 8-10, wherein the inner elongated hollow tubular member (13) is at a distal end thereof provided with said distally facing circular cutting edge (11).

12. Kit of parts according to any one of claims 1-11, wherein the outer elongated hollow tubular member (14) comprises a hollow metallic wire rope.

13. Kit of parts according to any one of claims 1-12, wherein the base member (10), which preferably comprises an inner elongated hollow tubular member (13), has at a proximal end thereof a connector (15) for connection, preferably releasable connection, to a motor (31), the connector (15) being capable of transferring said movement (LF, LB) along the central geometrical axis (A) and said rotation (ω) and torque (T).

14. The kit of parts according to any one of the previous claims, configured such that the core (51) is separated from the tissue (50) by shearing and/or tensile forces.

## Patentansprüche

1. Teilesatz, umfassend
ein Biopsieinstrument (1), und
eine Manövriereinheit (30), die einen Motor (31) umfasst, wobei das Biopsieinstrument (1) umfasst
ein äußeres längliches Hohlröhrenelement (14), das sich von einem proximalen Ende (14a) zu einem distalen Ende (14b) hin entlang einer zentralen geometrischen Achse (A) erstreckt, und
ein Basiselement (10), das sich von einem proximalen Ende (10a) zu einem distalen Ende (10b) entlang der zentralen geometrischen Achse (A) erstreckt, wobei ein distaler Endabschnitt (10b') des Basiselements (10) als ein längliches Hohlrohr (10') geformt ist, wobei das längliche Hohlrohr (10') am distalen Ende (10b) des Basiselements (10) dazu bestimmt ist, zumindest teilweise in ein Gewebe (50) eingeführt zu werden, aus dem eine Biopsie erhalten werden soll,
wobei das Basiselement (10) innerhalb des äußeren länglichen Hohlröhrenelements (14) angeordnet ist und relativ zu dem äußeren länglichen Hohlröhrenelement (14) unabhängig drehbar und translatorisch beweglich ist,
wobei das Basiselement (10) in der Lage ist, eine Kraft entlang der zentralen geometrischen Achse (A) so zu übertragen, dass eine Bewegung (LF, LB) des proximalen Endes (10a) des Basiselements (10) entlang der zentralen geometrischen Achse (A) in eine Bewegung (LF, LB) des distalen Endes (10b) des Basiselements (10) entlang der zentralen geometrischen Achse (A) übertragen wird, und ein Drehmoments um die zentrale geometrische Achse (A) so zu übertragen, dass eine Drehung (ω) und ein Drehmoment (T), das durch einen Motor (31) am proximalen Ende (10a) des Basiselements (10) um die zentrale geometrische Achse (A) angelegt wird, vom proximalen Ende (10a) des Basiselements (10) auf das distale Ende (10b) des Basiselements (10) übertragen wird, wodurch das distale Ende (10b) des Basiselements um die zentrale geometrische Achse (A) gedreht wird,
wobei das längliche Hohlrohr (10') in der Lage ist, durch eine Bewegung von (LF, LB) des proximalen Endes (10a) des Basiselements (10) entlang der zentralen geometrischen Achse (A) aus einem distalen Ende (14b) des äußeren länglichen Hohlröhrenelements (14) heraus vorgeschoben zu werden bzw. zurück in das äußere längliche Hohlröhrenelement (14) zurückgezogen zu werden, während es innerhalb und relativ zu dem äußeren länglichen Hohlröhrenelement (14) um die zentrale geometrische Achse (A) gedreht wird, indem der Motor (31) eine Drehung (ω) und ein Drehmoment (T) an dem proximalen Ende (10a) des Basiselements (10) ausübt,
wobei das längliche Hohlrohr (10') mit einer nach distal weisenden kreisförmigen Schneidkante (11) versehen ist, die eine Mundöffnung (10c) des distalen Endes (10b) des länglichen Hohlrohrs (10') definiert, und
wobei das längliche Hohlrohr (10') an einem distalen Abschnitt (10b') des länglichen Hohlrohrs (10') einen hohlen länglichen röhrenförmigen Probenaufnahmeabschnitt (10b') mit einer glatten Innenfläche (12) aufweist,
wobei das proximale Ende (10a) des Basiselements (10) dazu konfiguriert ist, mit dem Motor (31) verbunden zu werden, so dass eine Drehung (ω) und ein Drehmoment (T) durch den Motor (31) auf das proximale Ende (10a) des Basiselements (10) ausgeübt und durch das Basiselement (10) auf das längliche Hohlrohr (10) am distalen Ende (10b) des Basiselements (10) übertragen werden können, und wobei das Basiselement (10) flexibel ist und das äußere längliche Hohlröhrenelement (14) flexibel ist,
**dadurch gekennzeichnet, dass** der Motor (31) dazu ausgelegt ist, eine Drehung (ω) des länglichen Hohlrohrs (10') mit einer Drehzahl von mindestens 13 000 U/min, während das längliche Hohlrohr (10') aus dem distalen Ende (14b) des äußeren länglichen Hohlröhrenelements (14) vorgeschoben wird und zurück in das äußere längliche Hohlröhrenelement (14) zurückgezogen wird, innerhalb und relativ zu dem äußeren länglichen Hohlröhrenelement (14) um die zentrale geometrische Achse (A) bereitzustellen durch Anlegen der Drehung (ω) und des Drehmoments (T) auf das proximale Ende (10a) des Basiselements (10).

2. Teilesatz nach Anspruch 1, wobei die Drehzahl zwischen 13 000 U/min und 25 000 U/min, vorzugsweise zwischen 13 000 U/min und 20 000 U/min liegt.

3. Teilesatz nach Anspruch 1 oder 2, wobei die innere oder äußere Oberfläche des Probenaufnahmeabschnitts (10b') flüssigkeitsdicht ist, wobei vorzugsweise die gesamte Länge der inneren oder äußeren Oberflächen des Basiselements (10) flüssigkeitsdicht ist.

4. Teilesatz nach Anspruch 3, wobei die nach distal weisende kreisförmige Schneidkante (11) geformt ist und die glatte Innenfläche (12), vorzugsweise eine flüssigkeitsdichte glatte Innenfläche, mit der Schneidkante (11) verbunden ist, so dass sich die glatte Innenfläche (12), entlang der zentralen geometrischen Achse (A) gesehen, zu einem am weitesten distalen Teil der Schneidkante (11) erstreckt.

5. Teilesatz nach einem der Ansprüche 1-4, wobei die glatte Innenfläche (12) in einem solchen Ausmaß glatt ist, dass, wenn eine Referenzbiopsie erfasst werden soll, die Schneidkante (11) sowie das distale Ende (10b) des länglichen Hohlrohrs (10') dazu konfiguriert ist, entlang der zentralen geometrischen Achse (A) in ein Gewebe (50) vorgeschoben zu werden während es gedreht wird (ω, T), indem es an seinem proximalen Ende (10a) mit einer Drehzahl von mindestens 13 000 U/min vom Motor angetrieben wird, und dadurch einen Bohrkern (51) des Gewebes (50) ausschneidet, der aufgrund des Vorschubs (LF) des länglichen Hohlrohrs (10') relativ zum länglichen Hohlrohr (10') durch die Mundöffnung (10c) in den Probenaufnahmeabschnitt (10b') des länglichen Hohlrohrs (10') eintritt, wobei eine Umfangsaußenfläche des Bohrkerns (51) zumindest teilweise an der glatten Innenfläche (12) des Probenaufnahmeabschnitts (10b') anliegt, wobei, nach dem Zurückziehen des länglichen Hohlrohrs (10') aus dem Gewebe (50) während seines Drehens (ω, T) bei einer Drehzahl von mindestens 13 000 U/min, indem es an seinem proximalen Ende (10a) vom Motor angetrieben wird, dadurch der Bohrkern (51) des Gewebes (50) durch eine Zugkraft aufgrund des Zurückziehens (LB) des länglichen Hohlrohrs (10') und aufgrund einer an einer Grenzfläche zwischen der glatten Innenfläche (12) und der Umfangsaußenfläche des Bohrkerns (51) gebildeten Haftkraft, die den Bohrkern (51) im Inneren des Probenaufnahmeabschnitts (10b') mit der glatten Innenfläche (12) hält, vom Gewebe (50) losgelöst wird.

6. Teilesatz nach einem der Ansprüche 1-5, wobei die glatte Innenfläche eine Oberflächenrauhigkeit mit einem Ra-Wert von weniger als 1,5µm, vorzugsweise weniger als 1 µm, wenn sie aus Stahl, wie etwa einem Edelstahl medizinischer Qualität, gebildet ist, und weniger als 6 µm, wie zwischen 1 µm und 6 µm, wenn sie aus einem Material auf Polymerbasis gebildet ist, aufweist.

7. Teilesatz nach einem der Ansprüche 1-6, wobei die glatte Innenfläche (12) aus einem Material auf Polymerbasis gebildet ist.

8. Teilesatz nach einem der Ansprüche 1-7, wobei das Basiselement (10) ein inneres längliches Hohlröhrenelement (13) umfasst, das sich vom proximalen Ende (10a) zum distalen Ende (10b) des Basiselements (10) erstreckt.

9. Teilesatz nach den Ansprüchen 7 und 8, wobei das die glatte Innenfläche (12) bildende Material auf Polymerbasis als Folie, vorzugsweise als Schlauchfolie, bereitgestellt ist, die in das innere längliche Hohlröhrenelement (13) eingesetzt ist und die an einer Innenfläche des inneren länglichen Hohlröhrenelements (13) befestigt ist.

10. Teilesatz nach Anspruch 8 oder 9, wobei das innere längliche Hohlröhrenelement (13) ein hohles metallisches Drahtseil umfasst, das in der Lage ist, eine Kraft entlang der zentralen geometrischen Achse (A) so zu übertragen, dass eine Bewegung (LF, LB) des proximalen Endes (10a) entlang der zentralen geometrischen Achse (A) in eine Bewegung (LF, LB) des distalen Endes (10b) entlang der zentralen geometrischen Achse (A) übertragen wird, und ein Drehmoment um die zentrale geometrische Achse (A) so zu übertragen, dass eine Drehung (ω) und ein Drehmoment (T), die durch den Motor (31) am proximalen Ende (10a) um die zentrale geometrische Achse (A) angelegt werden, vom proximalen Ende (10a) zum distalen Ende (10b) übertragen werden, wodurch das distale Ende (10b) um die zentrale geometrische Achse (A) gedreht wird.

11. Teilesatz nach einem der Ansprüche 8-10, wobei das innere längliche Hohlröhrenelement (13) an seinem distalen Ende mit der nach distal weisenden kreisförmigen Schneidkante (11) versehen ist.

12. Teilesatz nach einem der Ansprüche 1-11, wobei das äußere längliche Hohlröhrenelement (14) ein hohles metallisches Drahtseil umfasst.

13. Teilesatz nach einem der Ansprüche 1-12, wobei das Basiselement (10), das vorzugsweise ein inneres längliches Hohlröhrenelement (13) umfasst, an seinem proximalen Ende einen Verbinder (15) zur Verbindung, vorzugsweise lösbaren Verbindung, mit einem Motor (31) aufweist, wobei der Verbinder (15) in der Lage ist, die Bewegung (LF, LB) entlang der zentralen geometrischen Achse (A) und die Drehung (ω) und das Drehmoment (T) zu übertragen.

14. Teilesatz nach einem der vorhergehenden Ansprüche, der so konfiguriert ist, dass der Bohrkern (51) durch Scher- und/oder Zugkräfte von dem Gewebe (50) abgetrennt wird.

## Revendications

1. Kit de pièces comprenant
un instrument de biopsie (1), et
une unité de manœuvre (30) comprenant un moteur (31), l'instrument de biopsie (1) comprenant
un élément tubulaire creux allongé extérieur (14) s'étendant d'une extrémité proximale (14a) au niveau d'une extrémité distale (14b) le long d'un axe géométrique central (A), et
un élément de base (10) s'étendant d'une extrémité proximale (10a) au niveau d'une extrémité distale (10b) le long de l'axe géométrique central (A), une partie d'extrémité distale (10b') de l'élément de base (10) étant conformée en un tube creux allongé (10'), le tube creux allongé (10') au niveau de l'extrémité distale (10b) de l'élément de base (10) étant destiné à être inséré au moins partiellement dans un tissu (50) à partir duquel une biopsie doit être obtenue,
l'élément de base (10) étant agencé à l'intérieur de l'élément tubulaire creux allongé extérieur (14) et étant mobile indépendamment en rotation et en translation par rapport à l'élément tubulaire creux allongé extérieur (14),
l'élément de base (10) étant apte à transférer une force le long de l'axe géométrique central (A) de telle sorte qu'un mouvement (LF, LB) de l'extrémité proximale (10a) de l'élément de base (10) le long de l'axe géométrique central (A) est transféré en un mouvement (LF, LB) de l'extrémité distale (10b) de l'élément de base (10) le long de l'axe géométrique central (A), et de transférer un couple autour de l'axe géométrique central (A) de telle sorte qu'une rotation (ω) et un couple (T) appliqués par un moteur (31) à l'extrémité proximale (10a) de l'élément de base (10) autour de l'axe géométrique central (A) sont transférés de l'extrémité proximale (10a) de l'élément de base (10) à l'extrémité distale (10b) de l'élément de base (10), faisant ainsi tourner l'extrémité distale (10b) de l'élément de base autour de l'axe géométrique central (A),
le tube creux allongé (10') étant apte à être avancé hors d'une extrémité distale (14b) de l'élément tubulaire creux allongé extérieur (14) et d'être rétracté dans l'élément tubulaire creux allongé extérieur (14) par un mouvement (LF, LB) de l'extrémité proximale (10a) de l'élément de base (10) le long de l'axe géométrique central (A), tout en étant tourné à l'intérieur et par rapport à l'élément tubulaire creux allongé extérieur (14) autour de l'axe géométrique central (A) par le moteur (31) appliquant une rotation (ω) et un couple (T) au niveau de l'extrémité proximale (10a) de l'élément de base (10),
le tube creux allongé (10') étant pourvu d'un bord coupant circulaire orienté distalement (11) définissant une embouchure (10c) de l'extrémité distale (10b) du tube creux allongé (10'), et
le tube creux allongé (10') présentant, au niveau d'une partie distale (10b') du tube creux allongé (10'), une partie d'acquisition d'échantillon tubulaire allongée creuse (10b') ayant une surface intérieure lisse (12),
l'extrémité proximale (10a) de l'élément de base (10) étant configurée pour être connectée au moteur (31) de sorte que la rotation (ω) et le couple (T) peuvent être appliqués par le moteur (31) à l'extrémité proximale (10a) de l'élément de base (10) et être transférés par l'élément de base (10) au tube creux allongé (10) au niveau de l'extrémité distale (10b) de l'élément de base (10), et l'élément de base (10) étant flexible et l'élément tubulaire creux allongé extérieur (14) étant flexible,
**caractérisé en ce que** le moteur (31) est configuré pour fournir une rotation (ω) du tube creux allongé (10'), à une vitesse de rotation d'au moins 13 000 tr/min, pendant que le tube creux allongé (10') est avancé hors de l'extrémité distale (14b) de l'élément tubulaire creux allongé extérieur (14) et est rétracté dans l'élément tubulaire creux allongé extérieur (14), à l'intérieur et par rapport à l'élément tubulaire creux allongé extérieur (14) autour de l'axe géométrique central (A) en appliquant une rotation (ω) et un couple (T) à l'extrémité proximale (10a) de l'élément de base (10).

2. Kit de pièces selon la revendication 1, la vitesse de rotation étant comprise entre 13 000 tr/min et 25 000 tr/min, de préférence entre 13 000 tr/min et 20 000 tr/min.

3. Kit de pièces selon la revendication 1 ou 2, la surface intérieure ou extérieure de la partie d'acquisition d'échantillon (10b') étant étanche aux liquides, toute la longueur des surfaces intérieure ou extérieure de l'élément de base (10) étant de préférence étanche aux liquides.

4. Kit de pièces selon la revendication 3, le bord coupant circulaire orienté distalement (11) étant conformé et la surface intérieure lisse (12), de préférence une surface intérieure lisse étanche aux liquides, se raccordant au bord coupant (11) de telle sorte que la surface intérieure lisse (12) s'étend, vue le long de l'axe géométrique central (A), jusqu'à une partie la plus distale du bord coupant (11).

5. Kit de pièces selon l'une quelconque des revendications 1 à 4, la surface intérieure lisse (12) étant lisse dans une mesure telle que lorsqu'une biopsie de référence doit être acquise, le bord coupant (11) et l'extrémité distale (10b) du tube creux allongé (10') sont configurés pour être avancés le long de l'axe géométrique central (A) dans un tissu (50) tout en étant mis en rotation (ω, T) par entraînement motorisé à son extrémité proximale (10a) à une vitesse de rotation d'au moins 13 000 tr/min et pour couper ainsi une carotte (51) du tissu (50) qui, en raison de l'avancement (LF) du tube creux allongé (10'), pénètre par rapport au tube creux allongé (10') à travers l'embouchure (10c) dans la partie d'acquisition d'échantillon (10b') du tube creux allongé (10') avec une surface extérieure circonférentielle de la carotte (51) venant au moins partiellement en butée contre la surface intérieure lisse (12) de la partie d'acquisition d'échantillon (10b'), après quoi le tube creux allongé (10') est rétracté du tissu (50) tout en étant tourné (ω, T) à une vitesse de rotation d'au moins 13 000 tr/min par entraînement motorisé à son extrémité proximale (10a), la carotte (51) du tissu (50) étant détachée du tissu (50) par une force de traction due à la rétraction (LB) du tube creux allongé (10') et due à une force adhésive formée à une interface entre la surface intérieure lisse (12) et la surface extérieure circonférentielle de la carotte (51), laquelle force maintient la carotte (51) à l'intérieur de la partie d'acquisition d'échantillon (10b') présentant la surface intérieure lisse (12).

6. Kit de pièces selon l'une quelconque des revendications 1 à 5, la surface intérieure lisse ayant une rugosité de surface avec une valeur Ra inférieure à 1,5 µm, de préférence inférieure à 1 µm, lorsqu'elle est formée d'acier, tel qu'un acier inoxydable de qualité médicale, et inférieure à 6 µm, telle qu'entre 1 µm et 6 µm, lorsqu'elle est formée d'un matériau à base de polymère.

7. Kit de pièces selon l'une quelconque des revendications 1 à 6, la surface intérieure lisse (12) étant formée d'un matériau à base de polymère.

8. Kit de pièces selon l'une quelconque des revendications 1 à 7, l'élément de base (10) comprenant un élément tubulaire creux allongé intérieur (13) s'étendant de l'extrémité proximale (10a) à l'extrémité distale (10b) de l'élément de base (10).

9. Kit de pièces selon les revendications 7 et 8, le matériau à base de polymère formant la surface intérieure lisse (12) étant fourni sous la forme d'un film, de préférence un film tubulaire, qui est inséré dans l'élément tubulaire creux allongé intérieur (13) et qui est fixé à une surface intérieure de l'élément tubulaire creux allongé intérieur (13).

10. Kit de pièces selon la revendication 8 ou 9, l'élément tubulaire creux allongé intérieur (13) comprenant un câble métallique creux apte à transférer une force le long de l'axe géométrique central (A) de telle sorte qu'un mouvement (LF, LB) de l'extrémité proximale (10a) le long de l'axe géométrique central (A) est transféré en un mouvement (LF, LB) de l'extrémité distale (10b) le long de l'axe géométrique central (A), et de transférer un couple autour de l'axe géométrique central (A) de telle sorte qu'une rotation (ω) et un couple (T) appliqués par le moteur (31) à l'extrémité proximale (10a) autour de l'axe géométrique central (A) sont transférés de l'extrémité proximale (10a) à l'extrémité distale (10b) faisant ainsi tourner l'extrémité distale (10b) autour de l'axe géométrique central (A).

11. Kit de pièces selon l'une quelconque des revendications 8 à 10, l'élément tubulaire creux allongé intérieur (13) étant, au niveau d'une extrémité distale de celui-ci, pourvu dudit bord coupant circulaire orienté distalement (11).

12. Kit de pièces selon l'une quelconque des revendications 1 à 11, l'élément tubulaire creux allongé extérieur (14) comprenant un câble métallique creux.

13. Kit de pièces selon l'une quelconque des revendications 1 à 12, l'élément de base (10), qui comprend de préférence un élément tubulaire creux allongé intérieur (13), comportant à une extrémité proximale de celui-ci un connecteur (15) pour une connexion, de préférence une connexion amovible, à un moteur (31), le connecteur (15) étant apte à transférer ledit mouvement (LF, LB) le long de l'axe géométrique central (A) et ladite rotation (ω) et ledit couple (T).

14. Kit de pièces selon l'une quelconque des revendications précédentes, configuré de telle sorte que la carotte (51) est séparée du tissu (50) par des forces de cisaillement et/ou de traction.
